# FASCICULE DE BREVET EUROPEEN

(11) **EP 1 992 329 B1**
(45) Date de publication et mention de la délivrance du brevet: **16.04.2014**
(21) Numéro de dépôt: 08155810.8
(22) Date de dépôt: 07.05.2008
(51) Int. Cl.: A61K 8/49, A61K 8/73, A61Q 5/10, A61K 8/41

(54) **Composition tinctoriale comprenant une base d'oxydation aminopyrazolopyridine et un polymère associatif particulier**
Färbezusammensetzung, die eine Oxidationsbasis aus Aminopyrazolopyridin und ein spezielles assoziatives Polymer enthält
Dyeing composition comprising an aminopyrazolopyridine oxidation base and a specific associative polymer

(30) Priorité: 09.05.2007 FR 0754951
(43) Date de publication de la demande: 19.11.2008
(73) Titulaire: L'Oréal, 75008 Paris (FR)
(72) Inventeur: Saunier, Jean-Baptiste, 75014, PARIS (FR)
(74) Mandataire: Fevrier, Murielle Françoise E.

(56) Documents cités:
- EP-A- 1 323 409
- EP-A- 1 473 023
- EP-A- 1 792 606
- WO-A-01/35917
- WO-A-02/076416
- WO-A-02/076419
- FR-A- 2 886 137
- US-A1- 2005 000 038

## Description

L'invention a pour objet une composition tinctoriale comprenant au moins une base aminopyrazolopyridine et un polymère associatif particulier. L'invention a aussi pour objet l'utilisation de cette composition pour la teinture des fibres kératiniques ainsi que le procédé de teinture mettant en oeuvre cette composition.

Il est connu de teindre les fibres kératiniques et en particulier les cheveux humains avec des compositions tinctoriales contenant des précurseurs de colorant d'oxydation, appelés généralement bases d'oxydation, tels que des ortho ou paraphénylènediamines, des ortho ou paraaminophénols et des composés hétérocycliques. Ces bases d'oxydation sont des composés incolores ou faiblement colorés qui, associés à des produits oxydants, peuvent donner naissance par un processus de condensation oxydative à des composés colorés.

On sait également que l'on peut faire varier les nuances obtenues avec ces bases d'oxydation en les associant à des coupleurs ou modificateurs de coloration, ces derniers étant choisis notamment parmi les métadiamines aromatiques, les métaaminophénols, les métadiphénols et certains composés hétérocycliques tels que des composés indoliques.

La variété des molécules mises en jeu au niveau des bases d'oxydation et des coupleurs, permet l'obtention d'une riche palette de couleurs.

La coloration dite "permanente" obtenue grâce à ces colorants d'oxydation, doit par ailleurs satisfaire un certain nombre d'exigences. Ainsi, elle doit être sans inconvénient sur le plan toxicologique, elle doit permettre d'obtenir des nuances dans l'intensité souhaitée et présenter une bonne tenue face aux agents extérieurs tels que la lumière, les intempéries, le lavage, les ondulations permanentes, la transpiration et les frottements.

Les colorants doivent également permettre de couvrir les cheveux blancs, et être enfin les moins sélectifs possibles, c'est-à-dire permettre d'obtenir des écarts de coloration les plus faibles possibles tout au long d'une même fibre kératinique, qui est en général différemment sensibilisée (i.e. abîmée) entre sa pointe et sa racine.

Il est déjà connu d'utiliser des bases d'oxydation aminopyrazolopyridine pour la teinture des fibres kératiniques, notamment dans la demande de brevet FR 2801308. Ces bases permettent d'obtenir des nuances variées.

Par ailleurs, il est connu d'utiliser dans le domaine de la coloration des fibres kératiniques des compositions de teinture mettant en oeuvre des polymères associatifs de type polyurethane, notamment dans le document FR2886137 et des polymères associatifs cellulosiques, notamment dans le document EP1323409

Le but de la présente invention est d'obtenir une composition pour la coloration des cheveux qui présente des propriétés tinctoriales améliorées en terme de puissance, de sélectivité et de résistance aux agents extérieurs.

Ce but est atteint avec la présente invention qui a pour objet une composition de coloration des fibres kératiniques comprenant, dans un milieu de teinture approprié,
- une ou plusieurs bases d'oxydation aminopyrazolopyridine choisie parmi les bases de formule (I) ou (II), 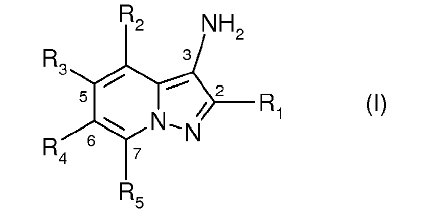 dans laquelle :
   - R₁, R₂, R₃, R₄ et R₅, identiques ou différents, représentent un atome d'hydrogène ou d'halogène ; un radical -NHSO₃H ; un radical hydroxyle ; un radical (C₁-C₄)alkyle ; un radical (C₁-C₄)alkoxy ; un radical (C₁-C₄)alkylthio ; mono(C₁-C₄)alkylamino ; un radical di(C₁-C₄)alkylamino dans lequel les deux groupements alkyle peuvent conjointement avec l'atome d'azote auquel ils sont liés, former un cycle pouvant être interrompu par un ou plusieurs atomes d'azote, d'oxygène ou de soufre ; un hétérocycle ; un radical nitro ; un radical phényle ; un radical carbonyle ; un radical alkoxy(C₁-C₄)carbonyle ; un radical carboxamido ; un radical cyano ; un radical amino ; un radical sulfonyle; un radical -CO₂H, un radical -SO₃H ; un radical -PO₃H₂ ; un radical -PO₄H₂; ou un groupement : 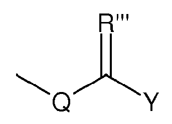 dans lequel R"' représente un atome d'oxygène ou d'azote, Q représente un atome d'oxygène, un groupement NH ou NH(C₁-C₄)alkyle, et Y représente un radical hydroxyle, amino, alkyle en C₁-C₄, (C₁-C₄)alkoxy, (C₁-C₄)alkylamino, ou di(C₁-C₄)alkylamino. 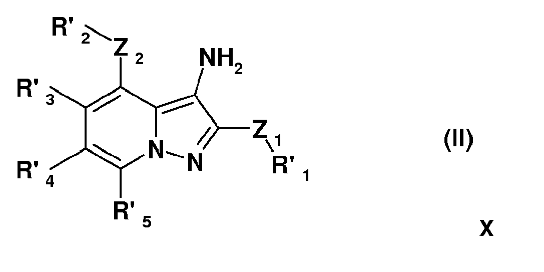 dans laquelle
      - Z₁ et Z₂ représentent indépendamment
         - une liaison covalente simple,
         - un radical divalent choisi parmi
         - un radical -O(CH₂)ₚ-, p désignant un nombre entier allant de 0 à 6
         - un radical -NR'₆(CH₂)_{q}(C₆H₄)ₜ-, q désignant un nombre entier allant de 0 à 6 et t désignant 0 ou 1. R'₆ représente un atome d'hydrogène, un radical alkyle en C1-C6 éventuellement substitué par un ou plusieurs groupements hydroxy.
      - Z₁ peut aussi représenter un radical divalent -S-, -SO-, -SO₂- lorsque R'₁ est un radical méthyle,
      - R'₁ et R'₂ représentent indépendamment :
         - un hydrogène
         - un radical alkyle en C₁-C₁₀, éventuellement substitué et éventuellement interrompu par un hétéroatome ou un groupement choisi parmi O, N, Si, S, SO, SO₂,
         - un halogène,
         - un radical SO₃H,
         - un cycle de 5 à 8 chaînons, substitué ou non, saturé, insaturé ou aromatique, renfermant éventuellement un ou plusieurs hétéroatomes ou groupements choisis parmi N, O, S, SO₂, -CO-, le cycle pouvant être cationique et/ou substitué par un radical cationique,
         - un groupement -N⁺R₁₇R₁₈R₁₉, R₁₇, R₁₈ et R₁₉ étant des alkyls linéaires ou ramifiés en C₁-C₅ éventuellement substituées par un ou plusieurs groupements hydroxy.
         lorsque Z₁ respectivement Z₂ représente une liaison covalente, alors R'₁ respectivement R'₂ peut aussi représenter un radical :
         - alkylcarbonyle en C₁-C₆, éventuellement substitué
         - -O-CO-R, -CO-O-R, NR-CO-R' ou -CO-NRR' dans lesquels R et R' représentent indépendamment un atome d'hydrogène ou un radical alkyle en C₁-C₆ éventuellement substitué,
      - R'₃, R'₄ et R'₅, identiques ou différents, représentent
         - un atome d'hydrogène,
         - un radical hydroxyle,
         - un radical alcoxy en C₁-C₆,
         - un radical alkylthio en C₁-C₆,
         - un radical amino,
         - un radical monoalkylamino,
         - un radical dialkylamino en C₁-C₆ dans lequel les radicaux alkyles peuvent former avec l'atome d'azote auquel ils sont rattachés un hétérocycle de 5 à 8 chaînons, saturé, insaturé, aromatique ou non, pouvant renfermer un ou plusieurs hétéroatomes ou groupements choisis parmi N, O, S, SO₂, CO, l'hétérocycle pouvant être cationique, et/ou substitué par un radical cationique,
         - un radical alkylcarbonyle en C₁-C₆, éventuellement substitué,
         - un radical -O-CO-R, -CO-O-R, NR-CO-R' ou -CO-NRR' avec R et R' tels que définis précédemment,
         - un halogène,
         - un radical -NHSO₃H,
         - un radical alkyle en C₁-C₄ éventuellement substitué,
         - un cycle carboné saturé, insaturé ou aromatique, éventuellement substitué.
         - R'₃, R'₄ et R'₅, peuvent former deux à deux un cycle partiellement saturé ou non,
      - X représente un ion ou groupe d'ions permettant d'assurer l'électronégativité du dérivé de formule (II),
      avec la condition qu'au moins un des groupements R'₁ et R'₂ représente un radical cationique.
- un ou plusieurs coupleurs,
- un ou plusieurs polymères associatifs choisis parmi les polymères associatifs cellulosiques.

L'invention a aussi pour objet un procédé de teinture mettant en oeuvre cette composition.

Un autre objet de l'invention est l'utilisation de la composition de la présente invention pour la teinture des fibres kératiniques, en particulier les fibres kératiniques humaines telles que les cheveux.

La composition de la présente invention permet en particulier d'obtenir une composition de coloration de fibres kératiniques qui conviennent pour une utilisation en coloration d'oxydation et permettent d'obtenir une coloration aux nuances variées, puissantes, esthétiques, peu sélectives et résistant bien aux diverses agressions que peuvent subir les cheveux tels que les shampooings, la lumière, la sueur et les déformations permanentes.

Il est à noter que dans ce qui va suivre, et à moins d'une autre indication, les bornes d'un domaine de valeurs sont comprises dans ce domaine.

Dans les composés de formule (I) à (II) ci-dessus, et sauf autre indication l'expression alkyle utilisée pour les radicaux alkyle ainsi que pour les groupements comportant une partie alkyle, signifie une chaîne carbonée, linéaire ou ramifiée, comportant de 1 à 4 atomes de carbone, substituée ou non substituée par un ou plusieurs hétérocycles, ou par un ou plusieurs groupements phényle ou par un ou plusieurs groupes choisis parmi les atomes d'halogène tel le chlore, le brome, l'iode et le fluor ; les radicaux hydroxyle, alcoxyle, amino, carbonyle, carboxamido, sulfonyle, -CO₂H, -SO₃H, -PO₃H₂, -PO₄H₂, -NHSO₃H, sulfonamide, monoalkyl(C₁-C₄)amino, trialkyl(C₁-C₄)ammonium, ou bien encore par un radical dialkyl(C₁-C₄)amino dans lequel les deux groupements alkyle peuvent former, conjointement avec l'atome d'azote dudit groupement dialkyl(C₁-C₄)amino auquel ils sont liés, un cycle pouvant être interrompu par un ou plusieurs atomes d'azote, d'oxygène ou de soufre.

De même, selon l'invention, l'expression alcoxy utilisée pour les radicaux alcoxy ainsi que pour les groupements comportant une partie alcoxy, signifie une chaîne O-carbonée, linéaire ou ramifiée, comportant de 1 à 4 de carbone, substituée ou non substituée par un ou plusieurs groupes choisis parmi les hétérocycles ; les atomes d'halogène tel le chlore, le brome, l'iode et le fluor ; les radicaux hydroxyle, amino, carbonyle, carboxamido, sulfonyle, -CO₂H, -SO₃H, -PO₃H₂, -PO₄H₂, -NHSO₃H, sulfonamide, monoalkyl(C₁-C₄)amino, trialkyl(C₁-C₄)ammonium, ou bien encore par un radical dialkyl(C₁-C₄)amino dans lequel les deux groupements alkyle peuvent former, conjointement avec l'atome d'azote dudit groupement dialkyl(C₁-C₄)amino auquel ils sont liés, un cycle pouvant être interrompu par un ou plusieurs atomes d'azote, d'oxygène ou de soufre.

Selon l'invention, on entend par hétérocycle, un cycle aromatique ou non contenant 5, 6, 7 ou 8 sommets, et de 1 à 3 hétéroatomes choisis parmi les atomes d'azote, de soufre et d'oxygène. Ces hétérocycles peuvent être condensés sur d'autres hétérocycles ou sur un groupement phényle. Ils peuvent être substitués par un atome d'halogène ; un radical (C₁-C₄)alkyle ; un radical (C₁-C₄)alkoxy ; un radical hydroxyle ; un radical amino ; un radical (C₁-C₄)alkylamino ; di(C₁-C₄) alkylamino dans lequel les deux groupements alkyle peuvent conjointement avec l'atome d'azote auquel ils sont liés, former un cycle pouvant être interrompu par un ou plusieurs atomes d'azote, d'oxygène ou de soufre. Ces hétérocycles peuvent, en outre, être quaternisés par un radical (C₁-C₄)alkyle.

Parmi ces hétérocycles éventuellement condensés, on peut notamment citer à titre d'exemple les cycles : thiadiazole, triazole, isoxazole, oxazole, azaphosphole, thiazole, isothiazole, imidazole, pyrazole, triazine, thiazine, pyrazine, pyridazine, pyrimidine, pyridine, diazépine, oxazépine, benzotriazole, benzoxazole, benzimidazole, benzothiazole, morpholine, pipéridine, pipérazine, azétidine, pyrrolidine, aziridine, 3-(2-hydroxyéthyl)benzothiazol-3-ium, et 1-(2-hydroxyéthyl)-pyridinium.

Selon l'invention, on entend par phényle, un radical phényle non substitué ou substitué par un ou plusieurs radicaux cyano, carbonyle, carboxamido, sulfonyle, - CO₂H, -SO₃H, -PO₃H₂, -PO₄H₂, hydroxyle, amino, monoalkyl(C₁-C₄)amino, ou dialkyl(C₁-C₄)amino dans lequel les deux groupements alkyle peuvent former, conjointement avec l'atome d'azote dudit groupement dialkyl(C₁-C₄)amino auquel ils sont liés, un cycle pouvant être interrompu par un ou plusieurs atomes d'azote, d'oxygène ou de soufre.

Parmi les groupements 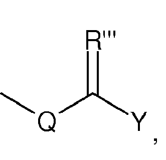 on peut notamment citer les groupement acétamide, diméthylurée, O-méthylcarbamate, méthylcarbonate, N-diméthylcarbamate et les esters.

Parmi les composés de formule (I) ci-dessus, on préfère les 3-amino pyrazolo-[1,5-a]-pyridines répondant à la sous-formule suivante, et leurs sels d'addition avec un acide ou avec une base : 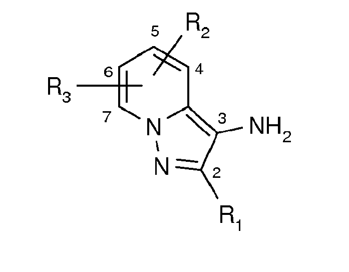 dans laquelle :
R₁, R₂, R₃, identiques ou différents, représentent un atome d'hydrogène ou d'halogène ; un radical hydroxyle ; un radical (C₁-C₄)alkyle ; un radical (C₁-C₄)alkylthio ; un radical (C₁-C₄)alkoxy ; un radical -NHSO₃H ; un radical amino ; un radical (C₁-C₄)alkylamino ; un radical di(C₁-C₄)alkylamino dans lequel les deux groupements alkyle peuvent conjointement avec l'atome d'azote auquel ils sont liés, former un cycle pouvant être interrompu par un ou plusieurs atomes d'azote, d'oxygène ou de soufre ; un hétérocycle tel que défini précédemment ; un radical sulfonamide, un radical carbonyle, un radical alcoxy(C₁-C₄)carbonyle, un radical carboxamido, ou un groupement de formule : 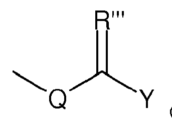
dans lequel R"' représente un atome d'oxygène ou d'azote, Q représente un atome d'oxygène, un groupement NH ou NH(C₁-C₄)alkyle, et Y représente un radical hydroxyle, amino, alkyle en C₁-C₄, (C₁-C₄)alkoxy, (C₁-C₄)alkylamino, ou di(C₁-C₄)alkylamino.

Parmi les 3-amino pyrazolo-[1,5-a]-pyridines de formule (I), utilisables à titre de base d'oxydation dans les compositions tinctoriales conformes à l'invention, on peut notamment citer :
- la pyrazolo[1,5-a]pyridin-3-ylamine ;
- la 2-acétylamino pyrazolo-[1,5-a] pyridin-3-ylamine ;
- la 2-morpholin-4-yl-pyrazolo[1 ,5-a]pyridin-3-ylamine ;
- l'acide 3-amino-pyrazolo[1 ,5-a]pyridin-2-carboxylique ;
- la 2-méthoxy-pyrazolo[1,5-a]pyridine-3-ylamino ;
- le (3-amino-pyrazolo[1 ,5-a]pyridine-7-yl)-méthanol ;
- le 2-(3-amino-pyrazolo[1 ,5-a]pyridine-5-yl)-éthanol ;
- le 2-(3-amino-pyrazolo[1,5-a]pyridine-7-yl)-éthanol ;
- le (3-amino-pyrazolo[1 ,5-a]pyridine-2-yl)-méthanol ;
- la 3,6-diamino-pyrazolo[1,5-a]pyridine ;
- la 3,4-diamino-pyrazolo[1,5-a]pyridine ;
- la pyrazolo[1,5-a]pyridine-3,7-diamine ;
- la 7-morpholin-4-yl-pyrazolo[1 ,5-a]pyridin-3-ylamine ;
- la pyrazolo[1,5-a]pyridine-3,5-diamine ;
- la 5-morpholin-4-yl-pyrazolo[1 ,5-a]pyridin-3-ylamine ;
- le 2-[(3-amino-pyrazolo[1 ,5-a]pyridin-5-yl)-(2-hydroxyéthyl)-amino]-éthanol ;
- le 2-[(3-amino-pyrazolo[1,5-a]pyridin-7-yl)-(2-hydroxyéthyl)-amino]-éthanol ;
- la 3-amino-pyrazolo[1,5-a]pyridine-5-ol ;
- la 3-amino-pyrazolo[1,5-a]pyridine-4-ol ;
- la 3-amino-pyrazolo[1 ,5-a]pyridine-6-ol ;
- la 3-amino-pyrazolo[1,5-a]pyridine-7-ol ;
- la 2-methoxy-6,7-dimethylpyrazolo[1,5-a]pyridin-3-amine ;
- la 2-[(3-aminopyrazolo[1,5-a]pyridin-2-yl)oxy]éthanol ;
- la 4-ethyl-2-methoxy-7-methylpyrazolo[1,5-a]pyridin-3-amine hydrochloride ;
- la 1-(3-aminopyrazolo[1,5-a]pyridin-2-yl)pyrrolidin-3-ol ;
- la 2,2'-[(3-aminopyrazolo[1 ,5-a]pyridin-2-yl)imino]diethanol ;
- la 2-[(3-aminopyrazolo[1,5-a]pyridin-2-yl)amino]ethanol ;
- la N2-(2-pyridin-3-ylethyl)pyrazolo[1 ,5-a]pyridine-2,3-diamine ;
et leurs d'addition avec un acide ou avec une base.
et leurs d'addition avec un acide ou avec une base.

Pour leur grande majorité, les 3-amino pyrazolo-[1,5-a]-pyridines de formule (I) sont des composés connus dans le domaine pharmaceutique, et sont décrites notamment dans le brevet US 5,457,200. Ces composés peuvent être préparés selon des méthodes de synthèse bien connues dans la littérature et telles que décrites par exemple dans le brevet US 5,457,200.

Par cycle ou hétérocycle cationique, on entend un cycle contenant un ou plusieurs groupements ammoniums quaternaires.

A titre d'exemple de radicaux du type -N⁺R₁₇R₁₈R₁₉, on peut citer les radicaux triméthylammonium, triéthylammonium, diméthyl-éthyl ammonium, diéthylméthylammonium, diisopropylméthylammonium, diéthyl-propyl ammonium, betahydroxyéthyl diéthyl ammonium, di (betahydroxyethyl)methylammonium, tri (betahydroxyethyl) ammonium.

A titre d'exemple d'hétérocycle cationique, on peut citer les hétérocycles imidazoliums, pyridiniums, pipéraziniums, pyrrolidiniums, morpholiniums, pyrimidiniums, thiazoliums, benzimidazoliums, benzothiazoliums, oxazoliums, benzotriazoliums, pyrazoliums, triazoliums, benzoxazoliums.

A titre d'exemple d'hétérocycle cationique, on peut citer les imidazoliums, les pyridiniums, les pipéraziniums, les pyrrolidiniums, les morpholiniums , les pyrimidiniums, les thiazoliums, les benzimidazoliums, les benzothiazoliums, les oxazoliums, les benzotriazoliums, les pyrazoliums, les triazoliums, les benzoxazoliums.

Les composés de formule (II) peuvent être éventuellement salifiés par des acides minéraux forts tels que par exemple HCl, HBr, Hl, H₂SO₄, H₃PO₄, ou des acides organiques tels que, par exemple, l'acide acétique, lactique, tartrique, citrique ou succinique, benzènesulfonique, para-toluènesulfonique, formique, méthanesulfonique.

S'ils possèdent des groupements anioniques tels que les groupements -CO₂H, -SO₃H, -PO₃H₂, -PO₄H₂, les composés de formules (I) peuvent être salifiés par des hydroxydes de métaux alcalins ou alcalinoterreux tels que la soude ou la potasse, par l'ammoniaque, par les amines organiques.

Ils peuvent aussi être sous forme de solvates par exemple un hydrate ou un solvate d'alcool linéaire ou ramifié tel que l'éthanol ou l'isopropanol.

Dans le cadre de l'invention, on entend par dérivé de formule (II) toutes formes mésomères ou isomères.

A titre d'exemples de dérivés de formule (II), on peut citer les composés suivants dans lesquels X⁻ est tel que défini précédemment :

| |
|---|
| 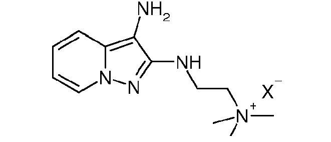 |
| Sel de [2-(3-Amino-pyrazolo[1,5-a]pyridin-2-ylamino)-ethyl]-trimethyl-ammonium |
| 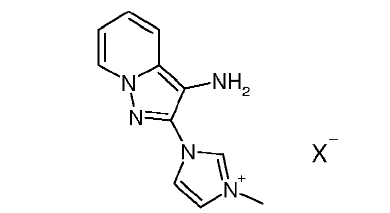 |
| Sel de 3-(3-Amino-pyrazolo[1,5-a]pyridin-2-yl)-1-methyl-3H-imidazol-1-ium |
| 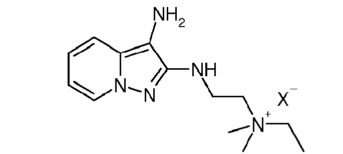 |
| Sel de [2-(3-Amino-pyrazolo[1,5-a]pyridin-2-ylamino)-ethyl]-ethyl-dimethyl-ammonium |
| 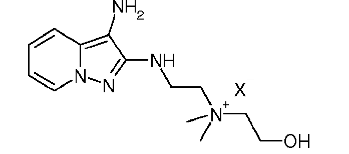 |
| Sel de [2-(3-Amino-pyrazolo[1,5-a]pyridin-2-ylamino)-ethyl]-(2-hydroxy-ethyl)-dimethyl-ammonium |
| 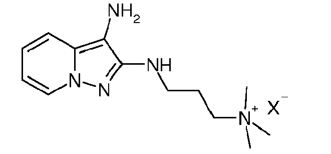 |
| Sel de [3-(3-Amino-pyrazolo[1 ,5-a]pyridin-2-ylamino)-propyl]-trimethyl-ammonium |
| 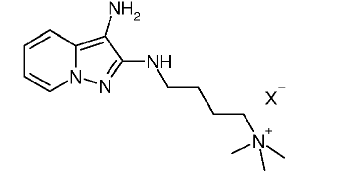 |
| Sel de [4-(3-Amino-pyrazolo[1,5-a]pyridin-2-ylamino)-butyl]-trimethyl-ammonium |
| 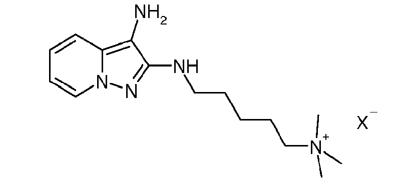 |
| Sel de [5-(3-Amino-pyrazolo[1,5-a]pyridin-2-ylamino)-pentyl]-trimethyl-ammonium |
| 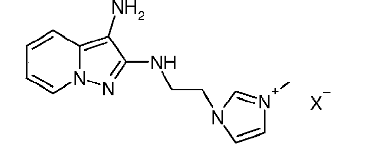 |
| Sel de 3-[2-(3-Amino-pyrazolo[1,5-a]pyridin-2-ylamino)-ethyl]-1-methyl-3H-imidazol-1-ium |
| 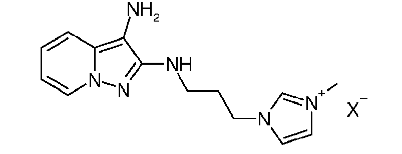 |
| Sel de 3-[3-(3-Amino-pyrazolo[1,5-a]pyridin-2-ylamino)-propyl]-1-methyl-3H-imidazol-1-ium |
| 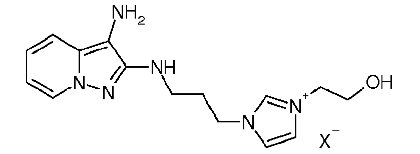 |
| Sel de 3-[3-(3-Amino-pyrazolo[1,5-a]pyridin-2-ylamino)-propyl]-1-(2-hydroxy-ethyl)-3H-imidazol-1-ium |
| 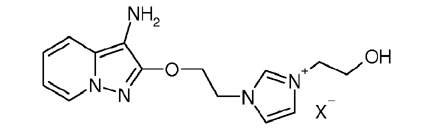 |
| Sel de 3-[2-(3-Amino-pyrazolo[1,5-a]pyridin-2-yloxy)-ethyl]-1-(2-hydroxy-ethyl)-3H-imidazol-1-ium |
| 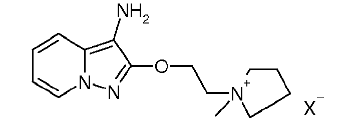 |
| Sel de 1-{2-[(3-aminopyrazolo[1,5-a]pyridin-2-yl)oxy]ethyl}-1-methylpyrrolidinium |
| 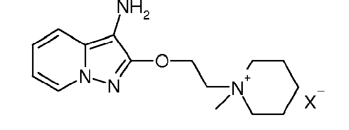 |
| Sel de 1-{2-[(3-aminopyrazolo[1,5-a]pyridin-2-yl)oxy]ethyl}-1-methylpiperidinium |
| 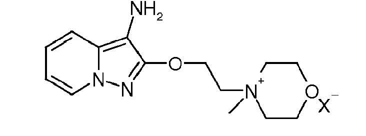 |
| Sel de 4-{2-[(3-aminopyrazolo[1,5-a]pyridin-2-yl)oxy]ethyl}-4-methylmorpholin-4-ium |
| 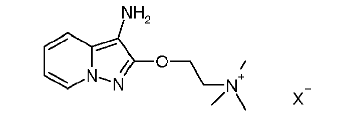 |
| Sel de {2-[(3-aminopyrazolo[1,5-a]pyhdin-2-yl)oxy]ethyl}-trimethyl-ammonium |
| 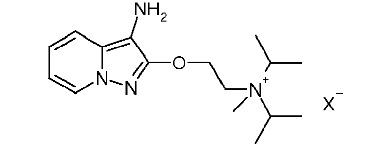 |
| Sel de {2-[(3-aminopyrazolo[1,5-a]pyridin-2-yl)oxy]ethyl}-diisopropyl-methyl-ammonium |
| 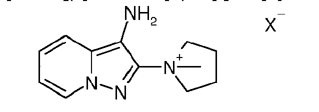 |
| Sel de 1-(3-aminopyrazolo[1,5-a]pyridin-2-yl)-1-methylpyrrolidinium |
| 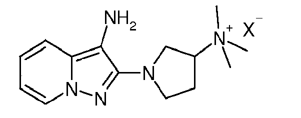 |
| Sel de [1-(3-Amino-pyrazolo[1,5-a]pyridin-2-yl)-pyrrolidin-3-yl]-trimethyl-ammonium |
| 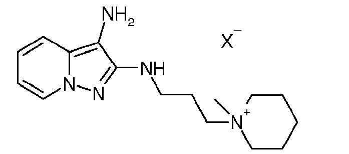 |
| Sel de 1-[3-(3-Amino-pyrazolo[1,5-a]pyridin-2-ylamino)-propyl]-1-methylpiperidinium |
| 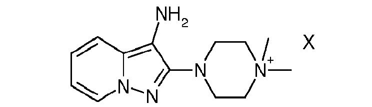 |
| Sel de 4-(3-Amino-pyrazolo[1,5-a]pyridin-2-yl)-1,1-dimethyl-piperazin-1-ium |
| 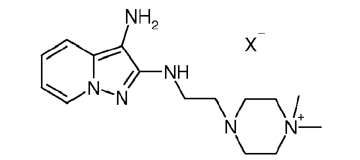 |
| Sel de 4-[2-(3-Amino-pyrazolo[1,5-a]pyridin-2-ylamino)-ethyl]-1,1-dimethyl-piperazin-1-ium |
| 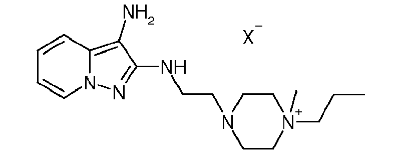 |
| Sel de 4-[2-(3-Amino-pyrazolo[1,5-a]pyridin-2-ylamino)-ethyl]-1-methyl-1-propyl-piperazin-1-ium |
| 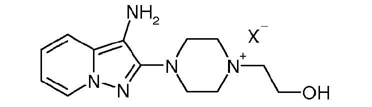 |
| Sel de 4-(3-Amino-pyrazolo[1,5-a]pyridin-2-yl)-1-(2-hydroxy-ethyl)-piperazin-1-ium |
| 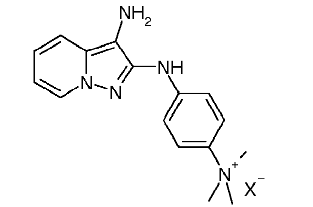 |
| Sel de [4-(3-Amino-pyrazolo[1,5-a]pyridin-2-ylamino)-phenyl]-trimethyl-ammonium |
| 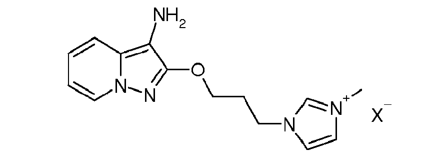 |
| Sel de 3-[3-(3-Amino-pyrazolo[1,5-a]pyridin-2-yloxy)-propyl]-1-methyl-3H-imidazol-1-ium |
| 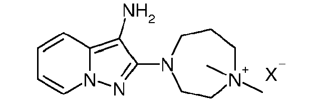 |
| Sel de 4-(3-Amino-pyrazolo[1,5-a]pyridin-2-yl)-1,1-dimethyl-[1,4]diazepan-1-ium |
| 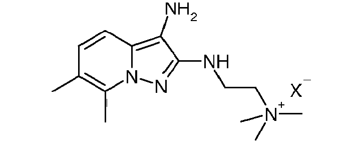 |
| Sel de [2-(3-Amino-6,7-di methyl-pyrazolo[1,5-a]pyridin-2-ylami no)-ethyl]-trimethyl-ammonium |
| 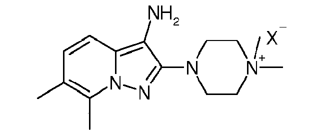 |
| Sel de 4-(3-Amino-6,7-dimethyl-pyrazolo[1,5-a]pyridin-2-yl)-1,1-dimethyl-piperazin-1-ium |
| 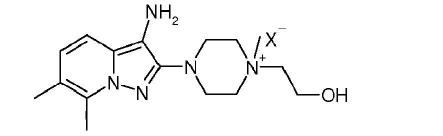 |
| Sel de 4-(3-Amino-6,7-dimethyl-pyrazolo[1,5-a]pyridin-2-yl)-1-(2-hydroxy-ethyl)-1-methyl-piperazin-1-ium |
| 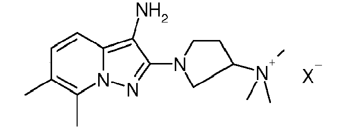 |
| Sel de [1-(3-Amino-6,7-dimethyl-pyrazolo[1,5-a]pyridin-2-yl)-pyrrolidin-3-yl]-trimethyl-ammonium |
| 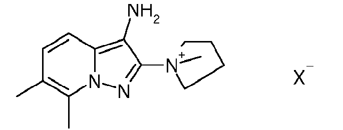 |
| Sel de 1-(3-amino-6,7-dimethylpyrazolo[1,5-a]pyridin-2-yl)-1-methylpyrrolidinium |
| 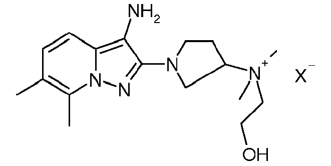 |
| Sel de [1-(3-Amino-6,7-dimethyl-pyrazolo[1,5-a]pyridin-2-yl)-pyrrolidin-3-yl]-(2-hydroxy-ethyl)-dimethyl-ammonium |
| 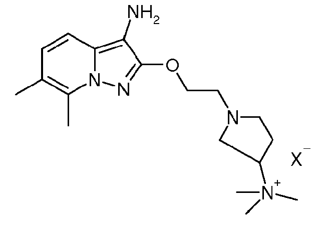 |
| Sel de {1-[2-(3-Amino-6,7-dimethyl-pyrazolo[1,5-a]pyridin-2-yloxy)-ethyl]-pyrrolidin-3-yl}-trimethyl-ammonium |
| 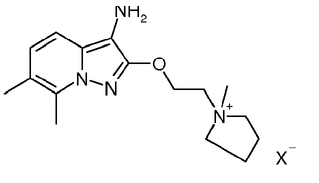 |
| Sel de 1-{2-[(3-amino-6,7-dimethylpyrazolo[1,5-a]pyridin-2-yl)oxy]ethyl}-1-methyl pyrrolidinium |
| 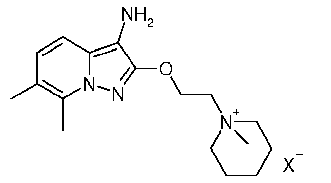 |
| Sel de 1-{2-[(3-amino-6,7-dimethylpyrazolo[1,5-a]pyridin-2-yl)oxy]ethyl}-1-methylpiperidinium |
| 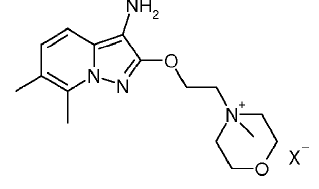 |
| Sel de 4-{2-[(3-amino-6,7-dimethylpyrazolo[1,5-a]pyridin-2-yl)oxy]ethyl}-4-methylmorpholin-4-ium |
| 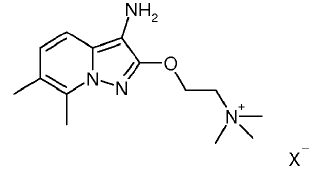 |
| Sel de {2-[(3-amino-6,7-dimethylpyrazolo[1,5-a]pyhdin-2-yl)oxy]ethyl}-thmethyl-ammonium |
| 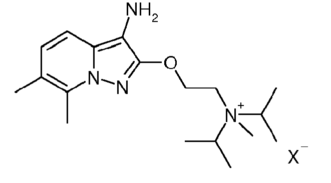 |
| Sel de {2-[(3-amino-6,7-dimethylpyrazolo[1,5-a]pyridin-2-yl)oxy]ethyl}-diisopropyl-methyl-ammonium |
| 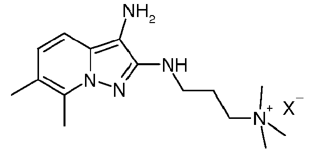 |
| Sel de [3-(3-Amino-6,7-dimethyl-pyrazolo[1,5-a]pyridin-2-ylamino)-propyl]-trimethyl-ammonium |
| 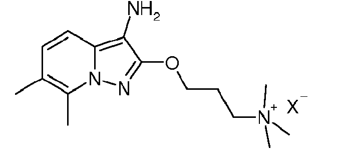 |
| Sel de [3-(3-Amino-6,7-dimethyl-pyrazolo[1,5-a]pyridin-2-yloxy)-propyl]-trimethyl-ammonium |
| 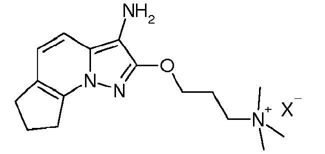 |
| Sel de [3-(3-Amino-7,8-dihydro-6H-cyclopenta[e]pyrazolo[1,5-a]pyridin-2-yloxy)-propyl]-trimethyl-ammonium |
| 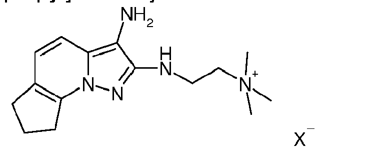 |
| Sel de {2-[(3-amino-7,8-dihydro-6H-cyclopenta[e]pyrazolo[1,5-a]pyridin-2-yl)amino]-ethyl}-trimethyl-ammonium |
| 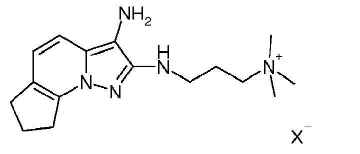 |
| Sel de {3-[(3-amino-7,8-dihydro-6H-cyclopenta[e]pyrazolo[1,5-a]pyridin-2-yl)amino]-propyl}-trimethyl-ammonium |
| 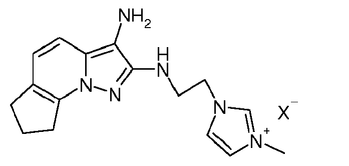 |
| Sel de 1-{2-[(3-amino-7,8-dihydro-6H-cyclopenta[e]pyrazolo[1,5-a]pyridin-2-yl)amino]ethyl}-3-methyl-1H-imidazol-3-ium |
| 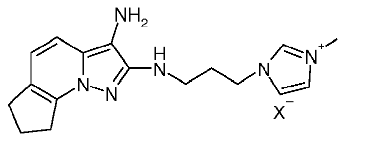 |
| Sel de 1-{3-[(3-amino-7,8-dihydro-6H-cyclopenta[e]pyrazolo[1,5-a]pyhdin-2-yl)amino]propyl}-3-methyl-1 H-imidazol-3-ium |
| 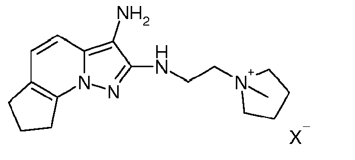 |
| Sel de 1-{2-[(3-amino-7,8-dihydro-6H-cyclopenta[e]pyrazolo[1,5-a]pyridin-2-yl)amino]ethyl}-1-methylpyrrolidinium |
| 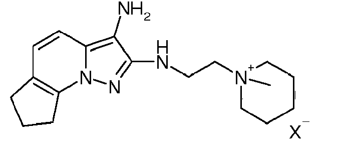 |
| Sel de 1-{2-[(3-amino-7,8-dihydro-6H-cyclopenta[e]pyrazolo[1,5-a]pyhdin-2-yl)amino]ethyl]-1-methylpiperidinium |
| 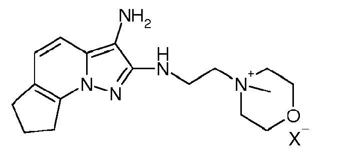 |
| Sel de 4-{2-[(3-amino-7,8-dihydro-6H-cyclopenta[e]pyrazolo[1,5-a]pyridin-2-yl)amino] ethyl]-4-methylmorpholin-4-ium |
| 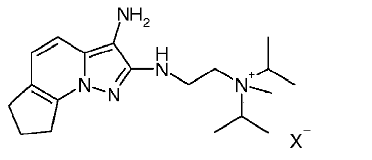 |
| Sel de 12-[(3-amino-7,8-dihydro-6H-cyclopenta[e]pyrazolo[1,5-a]pyridin-2-yl) amino] ethyl}-diisopropyl-methyl-ammonium |
| 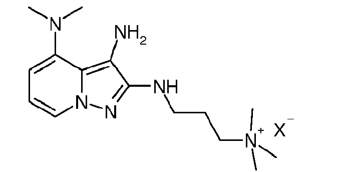 |
| Sel de [3-(3-Amino-4-dimethylamino-pyrazolo[1,5-a]pyridin-2-ylamino)-propyl]-trimethyl-ammonium |
| 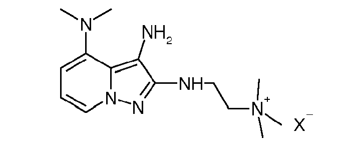 |
| Sel de [2-(3-Amino-4-dimethylamino-pyrazolo[1,5-a]pyridin-2-ylamino)-ethyl]-trimethyl-ammonium |
| 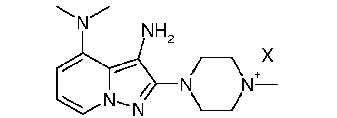 |
| Sel de 4-(3-Amino-4-dimethylamino-pyrazolo[1,5-a]pyridin-2-yl)-1-methyl-piperazin-1-ium |
| 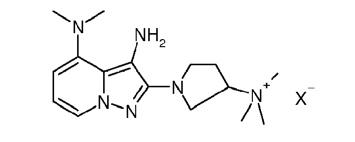 |
| Sel de [1-(3-Amino-4-dimethylamino-pyrazolo[1,5-a]pyridin-2-yl)-pyrrolidin-3-yl]-trimethyl-ammonium |
| 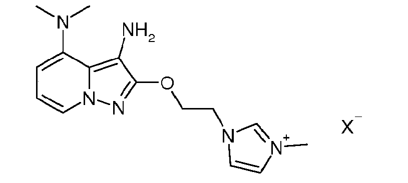 |
| Sel de 3-[2-(3-Amino-4-dimethylamino-pyrazolo[1,5-a]pyridin-2-yloxy)-ethyl]-1-methyl-3H-imidazol-1-ium |
| 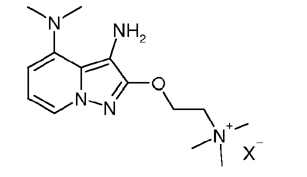 |
| Sel de [2-(3-Amino-4-dimethylamino-pyrazolo[1,5-a]pyridin-2-yloxy)-ethyl]-trimethyl-ammonium |
| 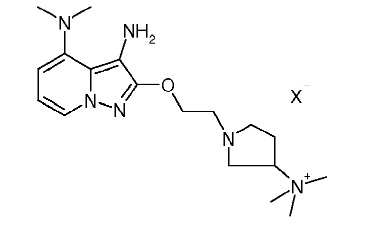 |
| Sel de {1-[2-(3-Amino-4-dimethylamino-pyrazolo[1,5-a]pyridin-2-yloxy)-ethyl]-pyrrolidin-3-yl)-trimethyl-ammonium |
| 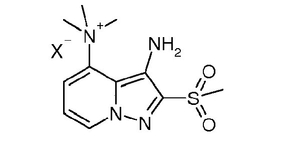 |
| Sel de (3-Amino-2-methanesulfonyl-pyrazolo[1,5-a]pyridin-4-yl)-trimethyl-ammonium |
| 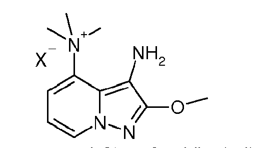 |
| Sel de (3-Amino-2-methoxy-pyrazolo[1,5-a]pyridin-4-yl)-trimethyl-ammonium |

La nature du contre-ion n'est pas déterminante sur le pouvoir tinctorial des composés de formule (II).

Lorsque R'₁ ou R'₂ désignent un hétérocycle, cet hétérocycle est de préférence un hétérocycle cationique ou un hétérocycle substitué par un radical cationique. A titre d'exemple, on peut citer les imidazoles substitués par un radical ammonium quaternaire ou les imidazoliums, les pipérazines substitués par un radical ammonium quaternaire ou les pipéraziniums, les pyrrolidines substitués par un radical ammonium quaternaire ou les pyrrolidiniums, les diazépanes substitués par un radical ammonium quaternaire ou les diazépaniums.

Selon un mode de réalisation différent, R'₁ ou R'₂ représentent un groupement - N⁺R₁₇R₁₈R₁₉, R₁₇, R₁₈ et R₁₉ étant des alkyls linéaires ou ramifiés en C₁-C₅ éventuellement substituées par un ou plusieurs groupements hydroxy, tel que trialkylammonium, tri(hydroxyalkyl)ammonium, hydroxyalkyl-dialkyl-ammonium ou di(hydroxyalkyl)alkylammonium.

Les radicaux R'₃, R'₄ et R'₅ indépendamment peuvent être un atome d'hydrogène, un radical alkyle en C₁-C₄ pouvant être substitué. A titre d'exemple, on peut citer les radicaux méthyle, éthyle, hydroxy éthyle, amino éthyle, propyle, butyle. Selon un mode de réalisation particulier, R'₃, R'₄ et R'₅ représentent indépendamment un atome d'hydrogène, un radical alkyle en C₁-C₄.

Selon un mode de réalisation particulier, R'₄ et R'₅ forment ensemble un cycle partiellement saturé ou insaturé à 5 ou 8 chaînons, notamment un cyclopentène ou cyclohexène, éventuellement substitué.

Selon un mode de réalisation particulier, le composé de formule (II) correspond à la formule (II') suivante : 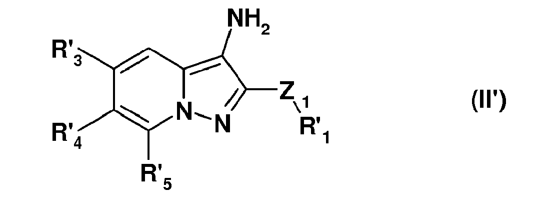 dans laquelle Z₁, R'₁, R'₃, R'₄ et R'₅ sont tels que définis précédemment.

Selon un mode de réalisation particulier de cette formule, Z₁ représente une liaison covalente, un radical -NR'₆(CH₂)_{q} ou un radical -O(CH₂)ₚ- et R'₁ est un radical cationique.

La ou les bases d'oxydation de l'invention sont en général présentes chacune en quantité comprise entre 0,001 à 10 % en poids environ du poids total de la composition tinctoriale, de préférence entre 0,005 et 6 %.

Les polymères associatifs sont des polymères de préférence hydrosolubles dont les molécules sont capables, en milieu aqueux, de s'associer réversiblement entre elles ou avec d'autres molécules pour conduire à un épaississement accru du milieu. L'invention contient aussi un polymère associatif cellulosique. Par composé cellulosique, on entend selon l'invention tout composé polysaccharidique possédant dans sa structure des enchaînements de résidus glucose unis par des liaisons β-1,4. Outre les celluloses non substituées, les dérivés de celluloses peuvent être anioniques, cationiques, amphotères ou non-ioniques. Parmi ces dérivés, on distingue les éthers de celluloses, les esters de celluloses et les esters éthers de celluloses. Parmi les esters de celluloses, on trouve les esters inorganiques de cellulose (nitrates, sulfates, ou phosphates de cellulose...), les esters organiques de cellulose (monoacétates, triacétates, amidopropionates, acétatebutyrates, acétatepropionates ou acétatetrimellitates de cellulose....) et les esters mixtes organique/inorganique de cellulose tels que les acétatebutyratesulfates et les acétatepropionatesulfates de cellulose. Parmi les esters éthers de cellulose, on peut citer les phtalates d'hydroxypropylméthylcellulose et les sulftates d'éthylcellulose.

Les composés cellulosiques de l'invention peuvent être choisis parmi les celluloses non substituées et les éthers de cellulose.

Parmi les éthers de cellulose non ioniques, on peut citer les alkylcelluloses telles que les méthylcelluloses et les éthylcelluloses ; les hydroxyalkylcelluloses telles que les hydroxyméthylcelluloses, les hydroxyéthylcelluloses et les hydroxypropylcelluloses ; les celluloses mixtes hydroxyalkyl-alkylcelluloses telles que les hydroxypropylméthylcelluloses, les hydroxyéthyl-méthylcelluloses, les hydroxyéthyl-éthylcelluloses et les hydroxybutyl-méthylcelluloses.

Parmi les éthers de cellulose anioniques, on peut citer les carboxyalkylcelluloses et leurs sels. A titre d'exemple, on peut citer les carboxyméthylcelluloses, les carboxyméthylméthylcelluloses et les carboxyméthylhydroxyéthylcelluloses et leurs sels de sodium.

Parmi les éthers de cellulose cationiques, on peut citer les hydroxyéthylcelluloses quaternisées réticulées ou non. Le quaternisant peut être notamment le chlorure de glycidyltriméthylammonium ou une amine grasse telle que la laurylamine ou la stéarylamine. Comme autre éther de cellulose cationique, on peut citer l'hydroxyéthylcellulosehydroxypropyltriméthylammonium.

Les dérivés cellulosiques utiles dans la présente invention peuvent être des dérivés de cellulose quaternisée à chaîne grasse, qui sont des polymères associatifs de type cationique.

Leur structure chimique comprend des zones hydrophiles, et des zones hydrophobes caractérisées par au moins une chaîne grasse.

Selon l'invention, on peut citer parmi eux et en particulier :
- les celluloses quaternisées modifiées par des groupements comportant au moins une chaîne grasse, tels que les groupes alkyle, arylalkyle, alkylaryle comportant de 8 à 30 atomes de carbone, ou des mélanges de ceux-ci,
- les hydroxyéthylcelluloses quaternisées modifiées par des groupements comportant au moins une chaîne grasse, tels que les groupes alkyle, arylalkyle, alkylaryle comportant de 8 à 30 atomes de carbone, ou des mélanges de ceux-ci.

Les radicaux aryle désignent de préférence les groupements phényle, benzyle, naphtyle ou anthryle.

On peut indiquer comme exemples d'alkylhydroxyéthylcelluloses quaternisées à chaînes grasses en C₈-C₃₀, les produits QUATRISOFT LM 200®, QUATRISOFT LM-X 529-18-A®, QUATRISOFT LM-X 529-18B® (alkyle en C₁₂) et QUATRISOFT LM-X 529-8® (alkyle en C₁₈) commercialisés par la société AMERCHOL et les produits CRODACEL QM®, CRODACEL QL® (alkyle en C₁₂) et CRODACEL QS® (alkyle en C₁₈) commercialisés par la société CRODA.

Une alkylhydroxyéthylcellulose quaternisée à chaîne grasse en C₈-C₃₀ particulièrement préférée selon l'invention est la laurylhydroxyéthylcellulose quaternisée.

A titre d'exemple d'éthers de cellulose cationiques utilisables dans les compositions selon la présente demande, on peut aussi citer ceux qui comprennent de 4 000 à 10 000 motifs anhydroglucose et sont substitués par au moins :
(a) un substituant de formule (IV) : [R₅₄R₅₅R₅₆R₅₉N⁺](X₅₂⁻)
   dans laquelle, R₅₄ et R₅₅ représentent indépendamment l'un de l'autre un groupe méthyle ou éthyle,
   R₅₆ représente un groupement alkyle, linéaire ou ramifié, en C₈-C₂₄ ou aralkyle dont la partie alkyle, linéaire ou ramifiée, est en C₈-C₂₄,
   R₅₉ représente un groupe divalent permettant le rattachement au groupement anhydroglucose et choisi parmi -(B)q₂-CH₂-CHOH-CH₂- ou -(B)q₂-CH₂-CH₂-,
   X₅₂⁻ représente un anion.
   q2 = 0 ou 1.
   B désigne un radical divalent (OCH₂CH₂)ₙ₂,
   n2 allant de 1 à 100,
(b) un substituant de formule (V) :

   [R₅₁R₅₂R₅₃R₅₈N⁺](X₅₁⁻)

   dans laquelle, R₅₁, R₅₂, R₅₃ représentent indépendamment l'un de l'autre un groupe méthyle ou éthyle ;
   R₅₈ représente un groupe divalent permettant le rattachement au groupement anhydroglucose et choisi parmi -(A)ₚ₂-CH₂-CHOH-CH₂- ou -CH₂CH₂-;
   X₅₁⁻ représente un anion.
   p2=0 ou 1.
   A désigne un radical divalent (OCH₂CH₂)ₙ₃,
   n3 allant de 1 à 100.

De préférence, le substituant (a) de formule (IV) est présent en une moyenne de 0,0003 à 0,08 moles, par mole d'unité anhydroglucose.

Les éthers de cellulose cationiques utilisables dans les compositions selon la présente demande sont de préférence des hydroxyéthyl ou hydroxypropyl celluloses.

Les éthers de cellulose cationiques utilisables dans les compositions selon la présente demande comprennent de préférence plus de 4 500, de manière encore préférée plus de 5000 et avantageusement plus de 6000 motifs anhydroglucose.

De préférence, les éthers de cellulose cationiques utilisables dans les compositions selon la présente demande comprennent de préférence jusqu'à 9000 et de manière préférée jusqu'à 8000 motifs anhydroglucose.

Ces éthers de cellulose cationiques et leur procédé de préparation sont décrits dans la demande WO 2005/000903, la partie de cette demande concernant les éthers de cellulose et leur procédé de préparation est incorporée par référence dans la présente demande.

Selon une variante préférée, les éthers de cellulose cationiques utilisables dans les compositions selon la présente demande sont formés de motifs (IX) et d'au moins un des motifs (VI), (VII), (VIII) suivants : 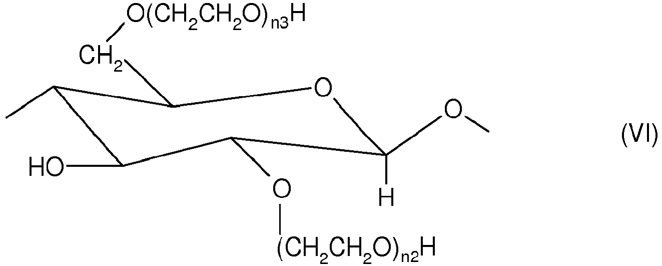 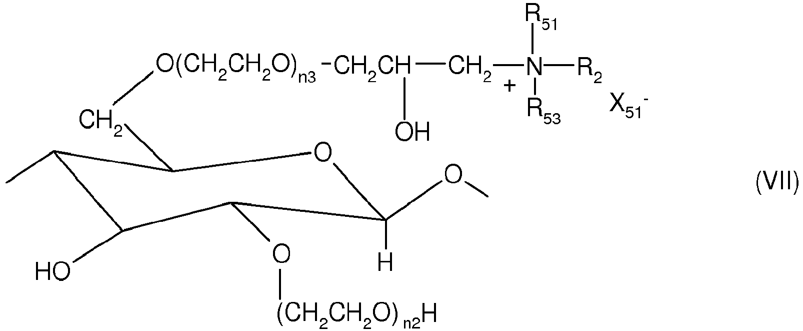 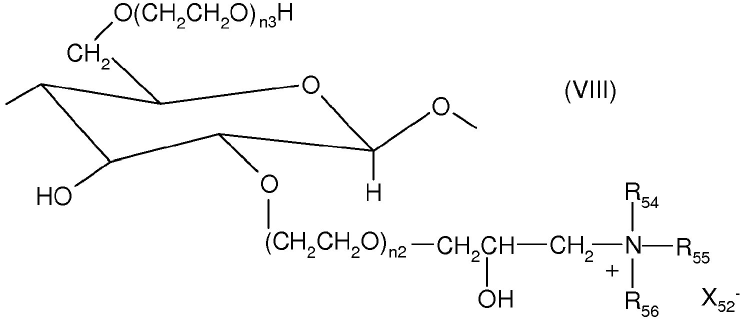 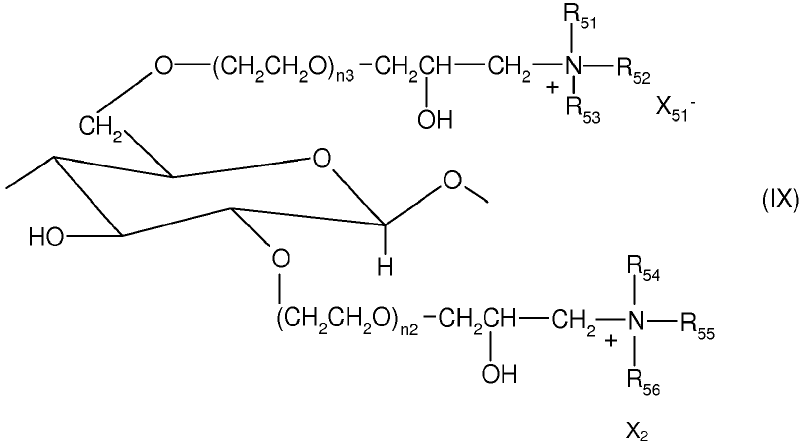 sous réserve que :
le nombre total des motifs (Vl)+(Vll)+(Vlll)+(IX) soit compris entre 4000 et 10000 ;
le rapport [(VIII) + (IXV)] / [(VI) +(VII) + (VIII) + (IX)] va de 0,0003 à 0,8 ;
le rapport [(VII) + (IX)] / [(VI) +(VII) + (VIII) + (IX)] va de 0,02 à 0,9 ;
n2 et n3, indépendamment l'un de l'autre, vont de 0 à 5.
R₅₁, R₅₂, R₅₃, R₅₄ et R₅₅ représentent, indépendamment l'un de l'autre, un groupe méthyle ou méthyle ;
R₅₆ représente un groupement alkyle en C₈-C₂₄ ou aralkyle dont la partie alkyle est en C₈-C₂₄, R₅₆ comprend de préférence de 10 à 24 atomes de carbone, de manière préférée de 12 à 24 atomes de carbone, et avantageusement de 12 à 15 atomes de carbone. Selon une variante particulière, R₅₆ est un groupement dodécyle linéaires ;
X₅₁⁻ et X₅₂⁻ représentent des amonts ; de préférence, ils sont choisis, indépendamment l'un de l'autre parmi les ions phosphate, nitrate, sulfate et halogénure (Cl⁻, Br⁻, F⁻, I⁻).

A titre d'éthers de cellulose cationiques utilisables, on peut citer les polymères de type SL-5, SL-30, SL-60 et SL-100 proposés par la société Amerchol.

La composition selon la présente demande peut comprendre un ou plusieurs éthers de cellulose cationiques selon l'invention.

Parmi les gommes de guar utilisées selon la présente invention, on peut citer :
- la gomme de guar hydroxypropylée vendue sous la dénomination "JAGUAR HP8" par la Société MEY HALL;
- la gomme de guar vendue sous la dénomination "GUARGEL D/15" par la SOCIETE FRANÇAISE DES COLLOIDES.

Parmi les gommes de cellulose non-ionique utilisées conformément à la présente invention, on peut mentionner :
- la méthylhydroxypropylcellulose vendue sous la dénomination "METHOCELF₄M STANDARD" par la Société DOW CHEMICAL;
- la méthylcellulose vendue sous la dénomination "METHYL CELLULOSE 200" par la Société LASERSON SABETAY;
- l'hydroxyéthylcellulose vendue sous la dénomination "NATROSOL HHR" par la Société AQUALON;
- l'hydroxypropylcellulose vendue sous la dénomination "KLUCEL H" par la Société AQUALON;
- la méthylhydroxyéthylcellulose vendue sous la dénomination "TYLOSE MH 300" par la Société HOECHST.

Dans la composition de la présente invention, le ou les polymères associatifs cellulosique sont présents en quantité comprise généralement de 0,00001 à 10 % en poids environ du poids total de la composition tinctoriale, préférentiellement de 0,001 à 5 % et encore plus préférentiellement de 0,001 à 3 % en poids environ de ce poids.

La composition tinctoriale de l'invention contient un ou plusieurs coupleurs conventionnellement utilisés pour la teinture de fibres kératiniques. Parmi ces coupleurs, on peut notamment citer les méta-phénylènediamines, les méta-aminophénols, les méta-diphénols, les coupleurs naphtaléniques, les coupleurs hétérocycliques et leur sels d'addition.

A titre d'exemple de coupleur, on peut citer le 2-méthyl 5-aminophénol, le 5-N-(β-hydroxyéthyl)amino 2-méthyl phénol, le 6-chloro-2-méthyl-5-aminophénol, le 3-amino phénol, le 2,4-dichloro-3-aminophenol, le 5-amino-4-chloro-o-cresol, le 1,3-dihydroxy benzène, le 1,3-dihydroxy 2-méthyl benzène, le 4-chloro 1,3-dihydroxy benzène, le 2,4-diamino 1-(β-hydroxyéthyloxy) benzène, le 2-amino 4-(β-hydroxyéthylamino) 1-méthoxybenzène, le 1,3-diamino benzène, le 1,3-bis-(2,4-diaminophénoxy) propane, la 3-uréido aniline, le 3-uréido 1-diméthylamino benzène, le sésamol, le 1-β-hydroxyéthylamino-3,4-méthylènedioxybenzène, l'α-naphtol, le 2 méthyl-1-naphtol, le 1,5-dihydroxy naphtalene, le 2,7-naphthalenediol, le 1-acetoxy-2-methylnaphthalene, le 6-hydroxy indole, le 4-hydroxy indole, le 4-hydroxy N-méthyl indole, la 2-amino-3-hydroxy pyridine, la 6- hydroxy benzomorpholine la 3,5-diamino-2,6-diméthoxypyridine, les 2,6-dihydroxy-3-4-dimethyl pyridine, le 3-amino-2-methylamino-6-methoxypyridine, le 1-N-(β-hydroxyéthyl)amino-3,4-méthylène dioxybenzène, le 2,6-bis-(β-hydroxyéthylamino)toluène, le 3-methyl-1-phenyl-5-pyrazolone phenyl methyl pyrazolone et leurs sels d'addition avec un acide.

La composition de la présente invention peut en outre comprendre une ou plusieurs bases d'oxydation additionnelles classiquement utilisées en teinture d'oxydation différentes des bases d'oxydation de formule (I). A titre d'exemple, ces bases d'oxydation additionnelles sont choisies parmi les para-phénylènediamines autres que celles décrites précédemment, les bis-phénylalkylènediamines, les para-aminophénols, les bis-para-aminophénols, les ortho-aminophénols, les bases hétérocycliques différentes des bases d'oxydation de formule (I) et leurs sels d'addition.

Parmi les para-phénylènediamines, on peut citer à titre d'exemple, la para-phénylènediamine, la para-toluènediamine, la 2-chloro para-phénylènediamine, la 2,3-diméthyl para-phénylènediamine, la 2,6-diméthyl para-phénylènediamine, la 2,6-diéthyl para-phénylènediamine, la 2,5-diméthyl para-phénylènediamine, la N,N-diméthyl para-phénylènediamine, la N,N-diéthyl para-phénylènediamine, la N,N-dipropyl para-phénylènediamine, la 4-amino N,N-diéthyl 3-méthyl aniline, la N,N-bis-(β-hydroxyéthyl) para-phénylènediamine, la 4-N,N-bis-(β-hydroxyéthyl)amino 2-méthyl aniline, la 4-N,N-bis-(β-hydroxyéthyl)amino 2-chloro aniline, la 2-β-hydroxyéthyl para-phénylènediamine, la 2-fluoro para-phénylènediamine, la 2-isopropyl para-phénylènediamine, la N-(β-hydroxypropyl) para-phénylènediamine, la 2-hydroxyméthyl para-phénylènediamine, la N,N-diméthyl 3-méthyl para-phénylènediamine, la N,N-(éthyl, β-hydroxyéthyl) para-phénylènediamine, la N-(β,γ-dihydroxypropyl) para-phénylènediamine, la N-(4'-aminophényl) para-phénylènediamine, la N-phényl para-phénylènediamine, la 2-β-hydroxyéthyloxy para-phénylènediamine, la 2-β-acétylaminoéthyloxy para-phénylènediamine, la N-(β-méthoxyéthyl) para-phénylènediamine, la 4-aminophénylpyrrolidine, la 2-thiényl para-phénylènediamine, le 2-β hydroxyéthylamino 5-amino toluène, la 3-hydroxy 1-(4'-aminophényl)pyrrolidine et leurs sels d'addition avec un acide.

Parmi les para-phénylènediamines citées ci-dessus, la para-phénylènediamine, la para-toluènediamine, la 2-isopropyl para-phénylènediamine, la 2-β-hydroxyéthyl para-phénylènediamine, la 2-β-hydroxyéthyloxy para-phénylènediamine, la 2,6-diméthyl para-phénylènediamine, la 2,6-diéthyl para-phénylènediamine, la 2,3-diméthyl para-phénylènediamine, la N,N-bis-(β-hydroxyéthyl) para-phénylènediamine, la 2-chloro para-phénylènediamine, la 2-β-acétylaminoéthyloxy para-phénylènediamine, et leurs sels d'addition avec un acide sont particulièrement préférées.

Parmi les bis-phénylalkylènediamines, on peut citer à titre d'exemple, le N,N'-bis-(β-hydroxyéthyl) N,N'-bis-(4'-aminophényl) 1,3-diamino propanol, la N,N'-bis-(β-hydroxyéthyl) N,N'-bis-(4'-aminophényl) éthylènediamine, la N,N'-bis-(4-aminophényl) tétraméthylènediamine, la N,N'-bis-(β-hydroxyéthyl) N,N'-bis-(4-aminophényl) tétraméthylènediamine, la N,N'-bis-(4-méthyl-aminophényl) tétraméthylènediamine, la N,N'-bis-(éthyl) N,N'-bis-(4'-amino, 3'-méthylphényl) éthylènediamine, le 1,8-bis-(2,5-diamino phénoxy)-3,6-dioxaoctane, et leurs sels d'addition avec un acide.

Parmi les para-aminophénols, on peut citer à titre d'exemple, le para-aminophénol, le 4-amino 3-méthyl phénol, le 4-amino 3-fluoro phénol, le 4-amino 3-hydroxyméthyl phénol, le 4-amino 2-méthyl phénol, le 4-amino 2-hydroxyméthyl phénol, le 4-amino 2-méthoxyméthyl phénol, le 4-amino 2-aminométhyl phénol, le 4-amino 2-(β-hydroxyéthyl aminométhyl) phénol, le 4-amino 2-fluoro phénol, le 1-hydroxy-4-methylamino-benzene, le 2-2'-methylenebis-4-amino phenol et leurs sels d'addition avec un acide.

Parmi les ortho-aminophénols, on peut citer à titre d'exemple, le 2-amino phénol, le 2-amino 5-méthyl phénol, le 2-amino 6-méthyl phénol, le 5-acétamido 2-amino phénol, et leurs sels d'addition avec un acide.

Parmi les bases hétérocycliques, on peut citer à titre d'exemple, les dérivés pyridiniques, les dérivés pyrimidiniques et les dérivés pyrazoliques.

Parmi les dérivés pyridiniques, on peut citer les composés décrits par exemple dans les brevets GB 1 026 978 et GB 1 153 196, comme la 2,5-diamino pyridine, la 2-(4-méthoxyphényl)amino 3-amino pyridine, la 2,3-diamino 6-méthoxy pyridine, la 2-(β-méthoxyéthyl)amino 3-amino 6-méthoxy pyridine, la 3,4-diamino pyridine, et leurs sels d'addition avec un acide.

Parmi les dérivés pyrimidiniques, on peut citer les composés décrits par exemple dans les brevets DE 2359399 ; JP 88-169571 ; JP 05-63124 ; EP 0770375 ou demande de brevet WO 96/15765 comme la 2,4,5,6-tétra-aminopyrimidine, la 4-hydroxy 2,5,6-triaminopyrimidine, la 2-hydroxy 4,5,6-triaminopyrimidine, la 2,4-dihydroxy 5,6-diaminopyrimidine, la 2,5,6-triaminopyrimidine, et les dérivés pyrazolo-pyrimidiniques tels ceux mentionnés dans la demande de brevet FR-A-2750048 et parmi lesquels on peut citer la pyrazolo-[1,5-a]-pyrimidine-3,7-diamine ; la 2,5-diméthyl pyrazolo-[1,5-a]-pyrimidine-3,7-diamine ; la pyrazolo-[1,5-a]-pyrimidine-3,5-diamine ; la 2,7-diméthyl pyrazolo-[1,5-a]-pyrimidine-3,5-diamine ; le 3-amino pyrazolo-[1,5-a]-pyrimidin-7-ol ; le 3-amino pyrazolo-[1,5-a]-pyrimidin-5-ol ; le 2-(3-amino pyrazolo-[1,5-a]-pyrimidin-7-ylamino)-éthanol, le 2-(7-amino pyrazolo-[1,5-a]-pyrimidin-3-ylamino)-éthanol, le 2-[(3-amino-pyrazolo[1,5-a]pyrimidin-7-yl)-(2-hydroxy-éthyl)-amino]-éthanol, le 2-[(7-amino-pyrazolo[1,5-a]pyrimidin-3-yl)-(2-hydroxy-éthyl)-amino]-éthanol, la 5,6-diméthyl pyrazolo-[1,5-a]-pyrimidine-3,7-diamine, la 2,6-diméthyl pyrazolo-[1,5-a]-pyrimidine-3,7-diamine, la 2, 5, N 7, N 7-tetraméthyl pyrazolo-[1,5-a]-pyrimidine-3,7-diamine, la 3-amino-5-méthyl-7-imidazolylpropylamino pyrazolo-[1,5-a]-pyrimidine et leurs sels d'addition avec un acide et leurs formes tautomères, lorsqu'il existe un équilibre tautomérique.

Parmi les dérivés pyrazoliques, on peut citer les composés décrits dans les brevets DE 3843892, DE 4133957 et demandes de brevet WO 94/08969, WO 94/08970, FR-A-2 733 749 et DE 195 43 988 comme le 4,5-diamino 1-méthyl pyrazole, le 4,5-diamino 1-(β-hydroxyéthyl) pyrazole, le 3,4-diamino pyrazole, le 4,5-diamino 1-(4'-chlorobenzyl) pyrazole, le 4,5-diamino 1,3-diméthyl pyrazole, le 4,5-diamino 3-méthyl 1-phényl pyrazole, le 4,5-diamino 1-méthyl 3-phényl pyrazole, le 4-amino 1,3-diméthyl 5-hydrazino pyrazole, le 1-benzyl 4,5-diamino 3-méthyl pyrazole, le 4,5-diamino 3-tert-butyl 1-méthyl pyrazole, le 4,5-diamino 1-tert-butyl 3-méthyl pyrazole, le 4,5-diamino 1-(P-hydroxyéthyl) 3-méthyl pyrazole, le 4,5-diamino 1-éthyl 3-méthyl pyrazole, le 4,5-diamino 1-éthyl 3-(4'-méthoxyphényl) pyrazole, le 4,5-diamino 1-éthyl 3-hydroxyméthyl pyrazole, le 4,5-diamino 3-hydroxyméthyl 1-méthyl pyrazole, le 4,5-diamino 3-hydroxyméthyl 1-isopropyl pyrazole, le 4,5-diamino 3-méthyl 1-isopropyl pyrazole, le 4-amino 5-(2'-aminoéthyl)amino 1,3-diméthyl pyrazole, le 3,4,5-triamino pyrazole, le 1-méthyl 3,4,5-triamino pyrazole, le 3,5-diamino 1-méthyl 4-méthylamino pyrazole, le 3,5-diamino 4-(β-hydroxyéthyl)amino 1-méthyl pyrazole, et leurs sels d'addition avec un acide.

On peut aussi citer les diaminopyrazolinones décrites dans la demande de brevet FR2886137 et en particulier la 2,3-diamino-6,7-dihydro-1H,5H-pyrazol-1-one et ses sels.

D'une manière générale, les sels d'addition des bases d'oxydation et des coupleurs utilisables dans le cadre de l'invention sont notamment choisis parmi les sels d'addition avec un acide tels que les chlorhydrates, les bromhydrates, les sulfates, les citrates, les succinates, les tartrates, les lactates, les tosylates, les benzènesulfonates, les phosphates et les acétates et les sels d'addition avec une base telles que la soude, la potasse, l'ammoniaque, les amines ou les alcanolamines.

La composition tinctoriale conforme à l'invention peut en outre contenir un ou plusieurs colorants directs pouvant notamment être choisis parmi les colorants nitrés de la série benzénique, les colorants directs azoïques, les colorants directs méthiniques. Ces colorants directs peuvent être de nature non ionique, anionique ou cationique.

Le milieu approprié pour la teinture appelé aussi support de teinture comprend généralement de l'eau ou un mélange d'eau et au moins un solvant organique pour solubiliser les composés qui ne seraient pas suffisamment solubles dans l'eau. A titre de solvant organique, on peut par exemple citer les alcanols inférieurs en C₁-C₄, tels que l'éthanol et l'isopropanol ; les polyols et éthers de polyols comme le 2-butoxyéthanol, le propylèneglycol, le monométhyléther de propylèneglycol, le monoéthyléther et le monométhyléther du diéthylèneglycol, ainsi que les alcools aromatiques comme l'alcool benzylique ou le phénoxyéthanol, et leurs mélanges.

Les solvants sont, de préférence, présents dans des proportions de préférence comprises entre 1 et 40 % en poids environ par rapport au poids total de la composition tinctoriale, et encore plus préférentiellement entre 5 et 30 % en poids environ.

La composition tinctoriale conforme à l'invention peut également renfermer divers adjuvants utilisés classiquement dans les compositions pour la teinture des cheveux, tels que des agents tensio-actifs anioniques, cationiques, non-ioniques, amphotères, zwitterioniques ou leurs mélanges, des polymères anioniques, cationiques, non-ioniques, amphotères, zwitterioniques ou leurs mélanges, des agents épaississants minéraux ou organiques, et en particulier les épaississants associatifs polymères anioniques, cationiques, non ioniques et amphotères autres que ceux de l'invention, des agents antioxydants, des agents de pénétration, des agents séquestrants, des parfums, des tampons, des agents dispersants, des agents de conditionnement tels que par exemple des silicones volatiles ou non volatiles, modifiées ou non modifiées, des agents filmogènes, des céramides, des agents conservateurs, des agents opacifiants.

Les adjuvants ci dessus sont en général présents en quantité comprise pour chacun d'eux entre 0,01 et 20 % en poids par rapport au poids de la composition.

Bien entendu, l'homme de l'art veillera à choisir ce ou ces éventuels composés complémentaires de manière telle que les propriétés avantageuses attachées intrinsèquement à la composition de teinture d'oxydation conforme à l'invention ne soient pas, ou substantiellement pas, altérées par la ou les adjonctions envisagées.

Le pH de la composition tinctoriale conforme à l'invention est généralement compris entre 3 et 12 environ, et de préférence entre 5 et 11 environ. Il peut être ajusté à la valeur désirée au moyen d'agents acidifiants ou alcalinisants habituellement utilisés en teinture des fibres kératiniques ou bien encore à l'aide de systèmes tampons classiques.

Parmi les agents acidifiants, on peut citer, à titre d'exemple, les acides minéraux ou organiques comme l'acide chlorhydrique, l'acide orthophosphorique, l'acide sulfurique, les acides carboxyliques comme l'acide acétique, l'acide tartrique, l'acide citrique, l'acide lactique, les acides sulfoniques.

Parmi les agents alcalinisants on peut citer, à titre d'exemple, l'ammoniaque, les carbonates alcalins, les alcanolamines telles que les mono-, di- et triéthanolamines ainsi que leurs dérivés, les hydroxydes de sodium ou de potassium et les composés de formule (X) suivante : 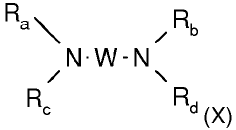 dans laquelle W est un reste propylène éventuellement substitué par un groupement hydroxyle ou un radical alkyle en C₁-C₄ ; Rₐ, R_{b}, R_{c} et R_{d}, identiques ou différents, représentent un atome d'hydrogène, un radical alkyle en C₁-C₄ ou hydroxyalkyle en C₁-C₄.

La composition tinctoriale selon l'invention peut se présenter sous des formes diverses, telles que sous forme de liquides, de crèmes, de gels, ou sous toute autre forme appropriée pour réaliser une teinture des fibres kératiniques, et notamment des cheveux humains.

Le procédé de la présente invention est un procédé dans lequel on applique sur les fibres la composition selon la présente invention telle que définie précédemment, en présence d'un agent oxydant pendant un temps suffisant pour développer la coloration désirée. La couleur peut être révélée à pH acide, neutre ou alcalin et l'agent oxydant peut être ajouté à la composition de l'invention juste au moment de l'emploi ou il peut être mis en oeuvre à partir d'une composition oxydante le contenant, appliquée simultanément ou séquentiellement à la composition de l'invention.

La révélation à pH acide peut s'avérer particulièrement intéressante.

Selon un mode de réalisation particulier, la composition selon la présente invention est mélangée, de préférence au moment de l'emploi, à une composition contenant, dans un milieu approprié pour la teinture, au moins un agent oxydant, cet agent oxydant étant présent en une quantité suffisante pour développer une coloration. Le mélange obtenu est ensuite appliqué sur les fibres kératiniques. Après un temps de pose de 3 à 50 minutes environ, de préférence 5 à 30 minutes environ, les fibres kératiniques sont rincées, lavées au shampooing, rincées à nouveau puis séchées.

Les agents oxydants classiquement utilisés pour la teinture d'oxydation des fibres kératiniques sont par exemple le peroxyde d'hydrogène, le peroxyde d'urée, les bromates de métaux alcalins, les persels tels que les perborates et persulfates, les peracides et les enzymes oxydases parmi lesquelles on peut citer les peroxydases, les oxydo-réductases à 2 électrons telles que les uricases et les oxygénases à 4 électrons comme les laccases. Le peroxyde d'hydrogène est particulièrement préféré.

La composition oxydante peut également renfermer divers adjuvants utilisés classiquement dans les compositions pour la teinture des cheveux et tels que définis précédemment.

Le pH de la composition oxydante renfermant l'agent oxydant est tel qu'après mélange avec la composition tinctoriale, le pH de la composition résultante appliquée sur les fibres kératiniques varie de préférence entre 3 et 12 environ, et encore plus préférentiellement entre 5 et 11. Il peut être ajusté à la valeur désirée au moyen d'agents acidifiants ou alcalinisants habituellement utilisés en teinture des fibres kératiniques et tels que définis précédemment.

La composition prête à l'emploi qui est finalement appliquée sur les fibres kératiniques peut se présenter sous des formes diverses, telles que sous forme de liquides, de crèmes, de gels ou sous toute autre forme appropriée pour réaliser une teinture des fibres kératiniques, et notamment des cheveux humains.

L'invention a aussi pour objet un dispositif à plusieurs compartiments ou "kit" de teinture dans lequel un premier compartiment renferme la composition tinctoriale de la présente invention définie ci-dessus et un deuxième compartiment renferme un agent oxydant. Ce dispositif peut être équipé d'un moyen permettant de délivrer sur les cheveux le mélange souhaité, tel que les dispositifs décrits dans le brevet FR-2 586 913 au nom de la demanderesse.

A partir de ce dispositif, il est possible de teindre les fibres kératiniques à partir d'un procédé qui comprend le mélange d'une composition tinctoriale comprenant une ou plusieurs bases d'oxydation de formule (I) et/ou (II), un ou plusieurs coupleurs et un ou plusieurs tensioactifs selon d'invention avec un agent oxydant, et l'application du mélange obtenu sur les fibres kératiniques pendant un temps suffisant pour développer la coloration désirée.

Les exemples qui suivent servent à illustrer l'invention sans toutefois présenter un caractère limitatif.

### Exemple 1

La composition 1 comprend les colorants suivants :

| En moles pour 100 g de composition | B2 | C2 |
|---|---|---|
| Composition 1 | 0.008 | 0.008 |

| | | |
|---|---|---|
| B2= Chlorhydrate de 2-[(3-aminopyrazolo[1,5-a]pyridin-2-yl)oxy]ethanol C2= 2-Chloro 6-méthyl 3-aminophénol introduits dans le milieu suivant : | | |

| | | |
|---|---|---|
| Mélange d'alcools linéaires en C₁₈ à C₂₄ (C₁₈/C₂₀/C₂₂/C₂₄ :7/57/30/6 - teneur en alcool > 95 %) | | 3.0 g |
| Alcool stéarylique oxyéthyléné (2 OE) | | 4.5 g |
| Alcool stéarylique oxyéthyléné (21 OE) | | 1.75 g |
| Acide oléique | | 2.6 g |
| Natrosol plus grade 330CS vendu par la société Aqualon | | 1.0 g |
| Acide polyacrylique réticulé | | 0.4 g |
| Hydroxypropyl méthyl cellulose | | 0.2 g |
| Monoéthalnolamide d'acide stéarique | | 3.0 g |
| Merquat 100 en solution aqueuse à 40% | | 4 g |
| Polymère cationique de formule (W) | | 2 g |
| Propylène glycol | | 2 g |
| Métabisulfite de sodium | | 0.71 g |
| EDTA (acide éthylènediamine tétra-acétique) | | 0.2 g |
| Ter-butyl hydroquinone | | 0.3 g |
| Monoéthanolamine | | 1 g |
| Ammoniaque à 20% de NH₃ | | 11 g |
| Parfum | q.s | |
| Eau déminéralisée | q.s.p | |

### Mode d'application

La composition est diluée extemporanément avec 1.5 fois son volume d'eau oxygénée à 25 volumes et dont le pH est de 3.

Le mélange ainsi réalisé est appliqué sur des cheveux gris naturels à 90% blancs, à raison de 30 g pour 3 g de cheveux pendant 30mn.

Les cheveux sont ensuite rincés, lavés avec un shampooing standard et séchés.

### Résultat

La coloration capillaire est évaluée de manière visuelle.

| | Hauteur de ton | Reflet |
|---|---|---|
| Composition 1 | Blond Foncé | Rouge violacé |

Cette nuance est tenace et uniforme

### Exemple 2

La composition 2 comprend les colorants suivants :

| En moles pour 100 g de composition | B3 | C3 |
|---|---|---|
| Composition 2 | 0.008 | 0.008 |

| | | |
|---|---|---|
| B3= Chlorhydrate de chlorure de 2-[(3-aminopyrazolo[1,5-a]pyridin-2-yl)amino]-N,N,N-trimethylethanaminium C3= 2-Méthyl 5-béta-hydroxyethylamino phénol | | |

### introduits dans le milieu suivant :

| | | |
|---|---|---|
| Alcool décylique oxyéthyléné à 12 moles O.E | | 7.5 g |
| Alcool oléocétylique oxyéthyléné à 30 moles O.E | | 6.0 g |
| Alcool laurique oxyéthyléné à 12 moles O.E | | 7.5 g |
| Acide laurique | | 2.5 g |
| Alcool cétylstéarylique (C₁₆/C₁₈ 50/50) | | 10.0 g |
| Acide polyacrylique réticulé (à 5 g% dans l'eau) | | 8.0 g |
| Silice pyrogénèe hydrophobe | | 1.0 g |
| Propylène glycol | | 10.0 g |
| Monoéthanolamine pure | | 5.0 g |
| Polymère SL-60 Amerchol | | 0.25 g |
| Monostéarate de glycérol | | 1.0 g |
| Thiolactate d'ammonium à 58% en solution aqueuse | | 0.8 g |
| Eau déminéralisée | q.s.p | 100 g |

### Mode d'application

La composition est diluée extemporanément avec 1.5 fois son poids d'eau oxygénée à 9 volumes et dont le pH est de 3.

Le mélange ainsi réalisé est appliqué sur des cheveux gris naturels à 90% blancs, à raison de 30 g pour 3 g de cheveux pendant 20mn.

Les cheveux sont ensuite rincés, lavés avec un shampooing standard et séchés.

### Résultats

La coloration capillaire est évaluée de manière visuelle.

| | Hauteur de ton | Reflet |
|---|---|---|
| Composition 2 | Blond Foncé | Violine Irisé Chromatique |

Cette nuance est tenace et uniforme

### Exemple 3

La composition 3 comprend les colorants suivants :

| En moles pour 100 g de composition | B4 | C4 |
|---|---|---|
| Composition 3 | 0.0055 | 0.0055 |

| | | |
|---|---|---|
| B4= Chlorhydrate de 2-[(3-aminopyrazolo[1,5-a]pyridin-2-yl)oxy]ethanol C4 = 2,4-Diaminophenoxyéthanol | | |

Cette composition colorante a été introduite dans le support suivant :

| | | |
|---|---|---|
| Alcool cétylstéarylique (C₁₆/C₁₈ 50/50) | | 16.2 g |
| Acide oléique | | 2.7 g |
| Alcool oléocétylique oxyéthyléné à 30 moles O.E | | 3.6 g |
| Alcool oléique | | 2.7 g |
| Oxyde de titane (anastase non traité enrobé de Polydiméthylsiloxane (98/2) | | 0.18 g |
| Polycondensat tetraméthyl héxaméthylènediamine / dichloro 1,3-propylène en solution aqueuse à 60% | | 6.66 g |
| Polymère SL-100 Amerchol | | 0.15 g |
| Monoéthanolamine pure | | 0.63 g |
| Métabisulfite de sodium | | 0.71 g |
| E.D.T.A | | 2.4 g |
| Ammoniaque à 20% de NH₃ | | 11 g |
| Eau déminéralisée | q.s.p | 100 g |

### Mode d'application

La composition est diluée extemporanément avec 1.5 fois son poids d'eau oxygénée à 20 volumes et dont le pH est de 3.

Le mélange ainsi réalisé est appliqué sur des cheveux gris naturels à 90% blancs, à raison de 30 g pour 3 g de cheveux pendant 15 mn.

Les cheveux sont ensuite rincés, lavés avec un shampooing standard et séchés.

### Résultats

La coloration capillaire est évaluée de manière visuelle.

| | Hauteur de ton | Reflet |
|---|---|---|
| Composition 3 | Blond Foncé | Irisé Rouge |

Cette nuance est tenace et uniforme

## Revendications

1. Composition tinctoriale comprenant, dans un milieu de teinture approprié, au moins une base d'oxydation aminopyrazolopyridine choisie parmi les bases de formule (I) ou (II), 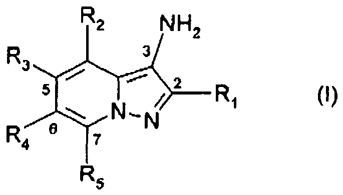 dans laquelle :
- R₁, R₂, R₃, R₄ et R₅, identiques ou différents, représentent un atome d'hydrogène ou d'halogène ; un radical -NHSO₃H ; un radical hydroxyle ; un radical (C₁-C₄)alkyle ; un radical (C₁-C₄)alkoxy ; un radical (C₁-C₄)alkylthio ; mono(C₁-C₄)alkylamino ; un radical di(C₁-C₄)alkylamIno dans lequel les deux groupements alkyle peuvent conjointement avec l'atome d'azote auquel Ils sont liés, former un cycle pouvant être interrompu par un ou plusieurs atomes d'azote, d'oxygène ou de soufre ; un hétérocycle ; un radical nitro ; un radical phényle ; un radical carbonyle ; un radical alkoxy(C₁-C₄)carbonyle ; un radical carboxamido ; un radical cyano ; un radical amino ; un radical sulfonyle; un radical -CO₂H, un radical -SO₃H ; un radical -PO₃H₂ ; un radical -PO₄H₂; ou un groupement : 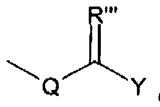 dans lequel R"' représente un atome d'oxygène ou d'azote, Q représente un atome d'oxygène, un groupement NH ou NH(C₁-C₄)alkyle, et Y représente un radical hydroxyle, amino, alkyle en C₁-C₄, (C₁-C₄)alkoxy, (C₁-C₄)alkylamino, ou di(C₁-C₄)alkylamino. 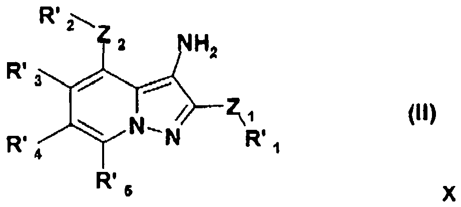
dans laquelle
• Z₁ et Z₂ représentent Indépendamment
- une liaison covalente simple.
- un radical divalent choisi parmi
- un radical -O(CH₂)ₚ-, p désignant un nombre entier allant de 0 à 6
- un radical -NR'₆(CH₂)_{q}(C₆H₄)ₜ-, q désignant un nombre entier allant de 0 à 6 et t désignant 0 ou 1. R'₆ représente un atome d'hydrogène, un radical alkyle en C₁-C₆ éventuellement substitué par un ou plusieurs groupements hydroxy.
• Z1 peut aussi représenter un radical divalent -S-, -SO-, -SO₂- lorsque R'₁ est un radical méthyle,
• R', et R'₂ représentent Indépendamment :
- un hydrogène
- un radical alkyle en C₁-C₁₀, éventuellement substitué et éventuellement interrompu par un hétéroatome ou un groupement choisi parmi O, N, Si, S,
SO, SO₂,
- un halogène,
- un radical SO₃H,
- un cycle de 5 à 8 chaînons, substitué ou non, saturé, insaturé ou aromatique, renfermant éventuellement un ou plusieurs hétéroatomes ou groupements choisis parmi N, O, S, SO₂, -CO-, le cycle pouvant être cationique et/ou substitué par un radical cationique,
- un groupement -N⁺R₁₇R₁₈R₁₉, R_{17,} R₁₈ et R₁₉ étant des alkyls linéaires ou ramifiés en C1-C5 éventuellement substituées par un ou plusieurs groupements hydroxy.
lorsque Z₁ respectivement Z₂ représente une liaison covalente, alors R'₁, respectivement R'₂ peut aussi représenter un radical :
- alkylcarbonye en C₁-C₆, éventuellement substitué
- -O-CO-R, -CO-O-R, NR-CO-R' ou -CO-NRR' dans lesquels R et R' représentent indépendamment un atome d'hydrogène ou un radical alkyle en C₁-C₆ éventuellement substitué,
• R'₃, R'₄ et R'₆, Identiques ou différents, représentent
- un atome d'hydrogène,
- un radical hydroxyle,
- un radical alcoxy en C₁-C₆,
- un radical alkylthio en C₁-C₆,
- un radical amino,
- un radical monoalkylamino,
- un radical dialkylamino en C₁-C₆ dans lequel les radicaux alkyles peuvent former avec l'atome d'azote auquel ils sont rattachés un hétérocycle de 5 à 8 chainons, saturé, insaturé, aromatique ou non, pouvant renfermer un ou plusieurs hétéroatomes ou groupements choisis parmi N, O, S, SO₂, CO, l'hétérocycle pouvant être cationique, et/ou substitué par un radical cationique,
- un radical alkyloarbonyle en C₁-C₆, éventuellement substitué,
- un radical -O-CO-R, -CO-O-R, NR-CO-R' ou -CO-NRR' avec R et R' tels que définis précédemment,
- un halogène,
- un radical -NHSO₃H,
- un radical alkyle en C₁-C₄ éventuellement substitué,
- un cycle carboné saturé, insaturé ou aromatique, éventuellement substitué.
- R'₃, R'₄ et R'₅, peuvent former deux à deux un cycle saturé ou non,
• X représente un Ion ou groupe d'ions permettant d'assurer félectronégativité du dérivé de formule (II),
avec la condition qu'au moins un des groupements R'₁ et R'₂ représente un radical cationique.
• un ou plusieurs coupleurs,
• un ou plusieurs polymères associatifs choisis parmi les polymères associatifs cellulosiques.

2. Composition selon la revendication 1, **caractérisée par le fait que** les composés de formule (I) sont choisis parmi les composés de sous-formule suivante, et leurs sels d'addition avec un acide ou avec une base : 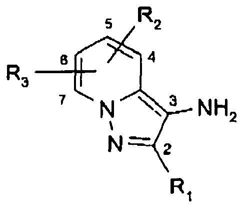 dans laquelle :
R₁, R₂, R₃, identiques ou différents, représentent un atome d'hydrogène ou d'halogène ; un radical hydroxyle ; un radical (C₁-C₄)alkyle ; un radical (C₁-C₄)alkylthio ; un radical (C₁-C₄)alkoxy ; un radical -NHSO₃H ; un radical amino ; un radical (C₁-C₄)alkylamino ; un radical di(C₁-C₄)alkylamino dans lequel les deux groupements alkyle peuvent conjointement avec l'atome d'azote auquel ils sont liés, former un cycle pouvant être interrompu par un ou plusieurs atomes d'azote, d'oxygène ou de soufre ; un hétérocycle ; un radical sulfonamide, un radical carbonyle, un radical alcoxy(C₁-C₄)carbonyle, un radical carboxamido, ou un groupement de formule suivante : 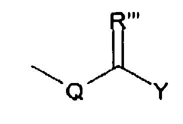 dans lequel R"' représente un atome d'oxygène ou d'azote, X représente un atome d'oxygène, un groupement NH ou NH(C₁-C₄)alkyle, et Y représente un radical hydroxyle, amino, alkyle en C₁-C₄, (C₁-C₄)alkoxy, (C₁-C₄)alkylamino, ou di(C₁-C₄)alkylamino,

3. Composition selon la revendication 1, **caractérisée par le fait que** les 3-amino pyrazolo-[1,5-a]-pyridines de formule (I) sont choisies parmi :
- la pyrazolo[1,5-a]pyridin-3-ylamine ;
- la 2-acétylamino pyrazolo-[1,5-a] pyridin-3-ylamine ;
- la 2-morpholin-4-yl-pyrazolo[1,5-a]pyridin-3-ylamine ;
- l'acide 3-amino-pyrazolo[1,5-a]pyridin-2-carboxylique ;
- la 2-méthoxy-pyrazolo[1,5-a]pyridine-3-ylamino ;
- le (3-amino-pyrazolo[1,5-a]pyridine-7-yl)-méthanol ;
- le 2-(3-amino-pyrazolo[1,5-a]pyridine-5-yl)-éthanol ;
- le 2-(3-amino-pyrazolo[1,5-a]pyridine-7-yl)-éthanol ;
- le (3-amino-pyrazolo[1,5-a]pyridlne-2-yl)-méthanol ;
- la 3,6-diamino-pyrazolo[1,5-a]pyridine ;
- la 3,4-diamino-pyrazolo[1,5-a]pyridine ;
- la pyrazolo[1,5-alpyridine-3,7-diamine ;
- la 7-morpholin-4-yl-pyrazolo[1,5-a]pyridin-3-ylamine ;
- la pyrazolo[1,5-a]pyridine-3,5-diamine ;
- la 5-morpholin-4-yl-pyrazolo[1,5-a]pyridin-3-ylamine ;
- le 2-[(3-amino-pyrazolo[1,5-a]pyridin-5-yl)-(2-hydroxyéthyl)-amino]-éthanol ;
- le 2-[(3-amino-pyrazolo[1,5-a]pyridin-7-yl)-(2-hydroxyéthyl)-amino]-éthanol ;
- la 3-amino-pyrazolo[1,5-a]pyridine-5-ol ;
- la 3-amino-pyrazolo[1,5-a]pyridine-4-ol ;
- la 3-amino-pyrazolo[1,5-a]pyrldlne-6-ol ;
- la 3-amino-pyrazolo[1,5-a]pyridine-7-ol ;
et leurs d'addition avec un acide ou avec une base.

4. Composition selon la revendication 1 **caractérisé en ce que**, dans la formule (II), Z₁ et/ou Z₂ représente une liaison covalente, un radical -NR'₆(CH₂)_{q}- ou un radical -Q(CH₂)ₚ et R'₁ et/ou R'₂ est un radical cationique.

5. Composition selon la revendication 4 dans laquelle R'₁ ou R'₂ désignent les Imidazoles substitués par un radical ammonium quaternaire ou les imidazoliums, les pipérazines substitués par un radical ammonium quaternaire ou les pipéraziniums, les pyrrolidines substitués par un radical ammonium quaternaire ou les pyrrolidiniums, les diazépanes substitués par un radical ammonium quaternaire ou les diazépaniums..

6. Composition selon la revendication 4 dans laquelle R'₁ et R'₂ représentent indépendamment un atome d'hydrogène, ou un groupement trialkylammonium, tri(hydroxyalkyl)ammonium, hydroxyalkyl-dialkyl-ammonium ou di(hydroxyalkyl)alkylammonium.

7. Composition selon l'une quelconque des revendications précédentes dans laquelle les radicaux R'₃, R'₄ et R'₅ de la formule (II) représentent indépendamment un atome d'hydrogène, un radical alkyle en C₁-C₄ pouvant être substitué.

8. Composition selon l'une quelconque des revendications précédentes dans laquelle le composé de formule (II) correspond à 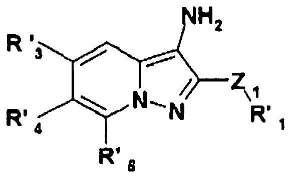 dans laquelle Z₁, R'₁, R'₃, R'₄ et R'₅ sont tels que définis à l'une quelconque des revendlcations précédentes.

9. Composition selon la revendication 8 dans laquelle Z₁ représente une liaison covalente, un radical -NR'₆(CH₂)_{q}- ou un radical -O(CH₂)ₚ- et R'₁ est un radical cationique.

10. Composition selon l'une quelconque des revendications précédentes dans laquelle le composé de formule (II) est choisi parmi :
| |
|---|
| 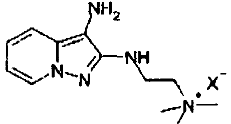 |
| Sel de [2,(3-Amino-pyrazolo[1,5-a]pyridin-2-ylamino)-ethyl]-trimethyl-ammonium |
| 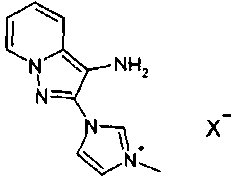 |
| Sel de 3-(3-Amino-pyrazolo[1,5-a]pyridin-2-yl)-1-methyl-3H-imidazol-1-ium |
| 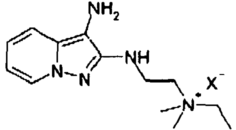 |
| Sel de [2-(3-Amino-pyrazolo[1,5-a]pyridin-2-ylamino)-ethyl]-ethyl-dimethyl-ammonium |
| 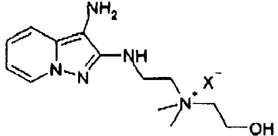 |
| Sel de [2-(3-Amino-pyrazolo[1,5-a]pyridin-2-ylamino)-ethyl]-(2-hydroxy-ethyl)-dimethyl-ammonium |
| 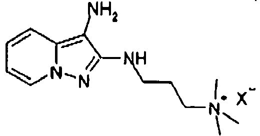 |
| Sel de [3-(3-Amino-pyrazolo[1,5-a]pyridin-2-ylamino)propyl]-trimethyl-ammonium |
| 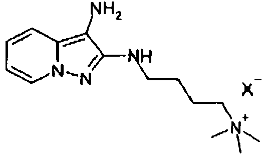 |
| Sel de [4-(3-Amino-pyrazolo[1,5-a]pyridin-2-ylamino)-butyl]-trimethyl-ammonium |
| 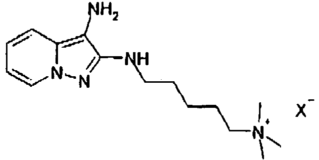 |
| Sel de [5-(3-Amino-pyrazolo[1,5-a]pyridin-2-ylamino)-pentyl]-trimethyl-ammonium |
| 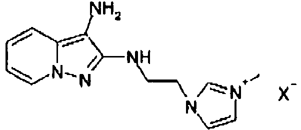 |
| Sel de 3-[2-(3-Amino-pyrozolo[1,5-a]pyridin-2-ylamino)-ethyl]-1-methyl-3H-imidazol-1-ium |
| 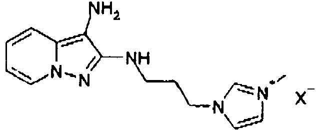 |
| Sel de 3-[3-(3-Amino-pyrazolo[1,5-a]pyridin-2-ylamino)-propyl]-1-methyl-3H-imidazol-1-ium |
| 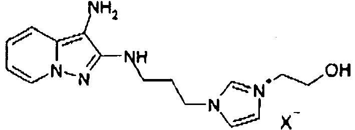 |
| Sel de 3-[3-(3-Amino-pyrazolo[1,5-a]pyridin-2-ylamino)-propyl]-1-2-(hydroxy-ethyl)-3H-imidazol-1-ium |
| 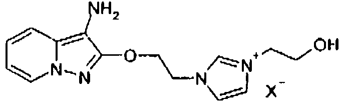 |
| Sel de 3-12-(3-Amino-pyrazolo[1,5-a]pyridin-2-yloxy)-ethyl]-1-(2-hydroxy-ethyl)-3H-imidazol-1-ium |
| 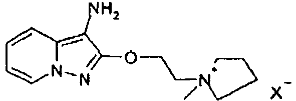 |
| Sel de 1-{2-[(3-aminopyrazolo[1,5-a]pyridin-2-yl)oxy]ethyl}-1-methylpyrrolidinium |
| 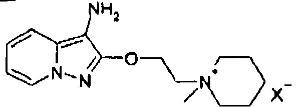 |
| Sel de 1-{2-[(3-aminopyrazolo[1,5-a]pyridin-2-yl]oxy]ethyl}-1-methylpiperidinium |
| 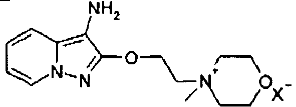 |
| Sel de 4-{2-[(3-aminopyrazolo[1,5-a]pyridin-2-yl)oxy]ethyl}-4-methylmorpholin-4-ium |
| 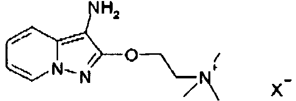 |
| Sel de {2-[(3-aminopyrazolo[1,5-a]pyridin-2-yl)oxy]ethyl}-trimethyl-ammonium |
| 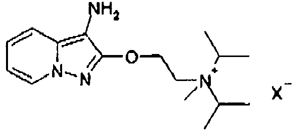 |
| Sel de {2-[(3-aminopyrazolor[1,5-a]pyridin-2-yl)oxy]ethyl}-dlisopropyl-methyl-ammonium |
| 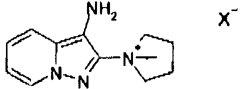 |
| Sel de 1-(3-aminopyrazolo[1,5-a]pyridn-2-yl)-1-methylpyrrolldinium |
| 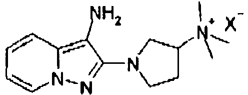 |
| Sel de [1-(3-Amino-pyrazolo[1,5-a]pyridin-2-yl)-pyrrolidin-3-yl]-trimethyl-ammonium |
| 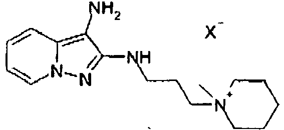 |
| Sel de 1-[3-(3-Amino-pyrazolo[1,5-a]pyridin-2-ylamino)-propyl]-1-methylpiperidinium |
| 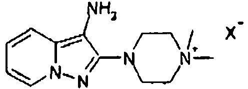 |
| Sel de 4-(3-Amino-pyrazolo[1,5-a]pyridin-2-yl)-1,1-dimethyl-piperazin-1-ium |
| 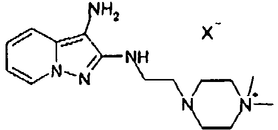 |
| Sel de 4-[2-(3-Amino-pyrazolo[1,5-a]pyridin-2-ylamino)-ethyl]-1,1-dimethyl-piperazin-1-ium |
| 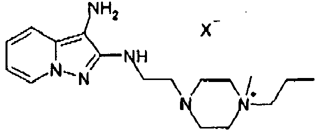 |
| Sel de 4-[2-(3-Amino-pyrazolo[1,5-a]pyridin-2-ylamino)-ethyl]-1-methyl-1-propyl-piperazin-1-ium |
| 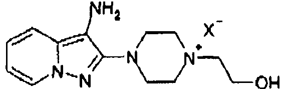 |
| Sel de 4-(3-Amino-pyrazolo[1,5-a]pyridin-2-yl)-1-(2-hydroxy-ethyl)-piperazin-1-ium |
| 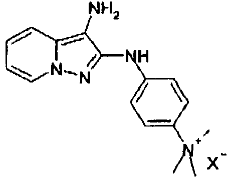 |
| Sel de [4-(3-Amino-pyrazolo[1,5-a]pyridin-2-ylamino)-phenyl]-trimethyl-ammonium |
| 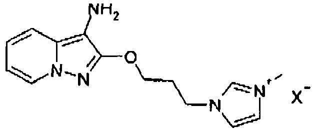 |
| Sel de 3-[3-(3-Amino-prazolo[1,5-a]pyridin-2-yloxy)-propyl]-1-methyl-3H-Imidazol-1-ium |
| 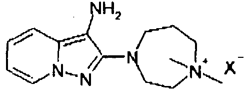 |
| Sel de 4-(3-Amino-pyrazolo[1,5-a]pyridin-2-yl)-1,1-dimethyl-[1,4]diazepan-1-ium |
| 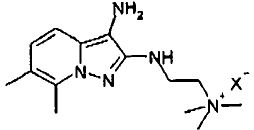 |
| Sel de [2-(3-Amino-6,7-dimethyl-pyrazolo[1,5-a]pyridin-2-ylamino)-ethyl]-trimethyl-ammonium |
| 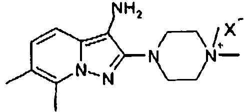 |
| Sel de 4-(3-Amino-6,7-dimethyl-pyrazolo[1,5-a]pyridin-2-yl)-1,1-dimethyl-piperazin-1-ium |
| 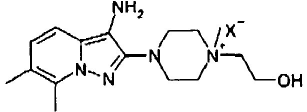 |
| Sel de 4-(3-Amino-6,7-dimethyl-pyrazolo[1,5-a]pyridin-2-yl)-1-(2-hydroxy-ethyl)-1-methyl-piperezin-1-ium |
| 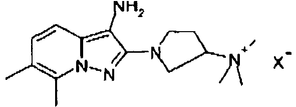 |
| Sel de [1-(3-Amino-6,7-dimethyl-pyrazolo[1,5-a]pyridin-2-yl)-pyrrolidin-3-yl]-trimethyl-ammonium |
| 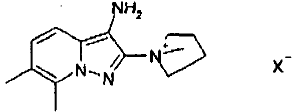 |
| Sel de 1-(3-amino-6,7-dimethylpyrazolo[1,5-a]pyridin-2-yl)-1-methylpyrrolidinium |
| 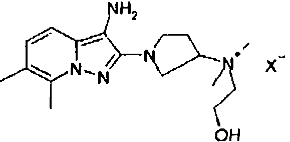 |
| Sel de [1-(3-Amino-6,7-dimethyl-purazolo[1,5-a]pyridin-3-yl]-(2-hydroxy-ethyl)-dimethyl-ammonium |
| 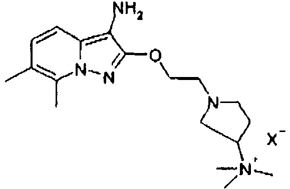 |
| Sel de {1-[2-(3-Amino-6,7-dimethyl-pyrazolo[1,5-a]pyridin-2-yloxy)-ethyl]-pyrrolidin-3-yl}-trimethyl-ammonium |
| 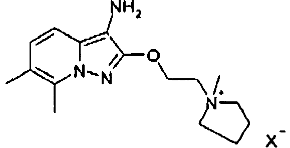 |
| Sel de 1-{2-[(3-amino-6,7-dimethylpyrazolo[1,5-a]pyridin-2-yl)oxy]ethyl}-1-methyl pyrrolidinium |
| 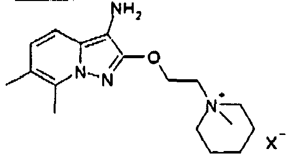 |
| Sel de 1-{2-[(3-amino-6,7-dimethylpyrazolo[1,5-a]pyridin-2-yl)oxy]ethyl}-1-methylpiperidinium |
| 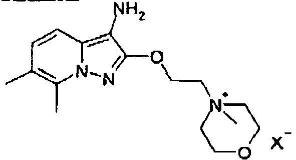 |
| Sel de 4-{2-[(3-amino-6,7-dimethylpyrazolo[1,5-a]pyridin-2-yl)oxy]ethyl}-4-methylmorpholin-4-ium |
| 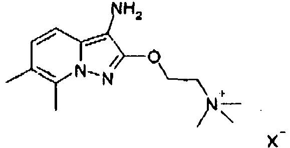 |
| Sel de {2-[(3.amino-6,7-dimethylpyrazolo[1,5-a]pyridin-2-yl)oxy]ethyl}-trimethyl-ammonium |
| 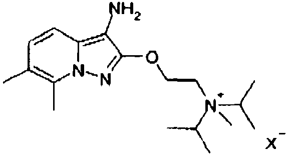 |
| Sel de {2-[(3-amino-6,7-dimethylpyrazolo[1,5-a]pyridin-2-yl)oxy]ethyl}-diisopropyl-methyl-ammonium |
| 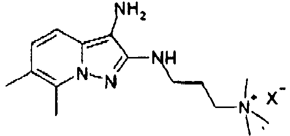 |
| Sel de 13-(3-Amino-6,7-dimethyl-pyrazolo[1,5-a]pyridin-2-ylamino)-propyl]-trimethyl-ammonium |
| 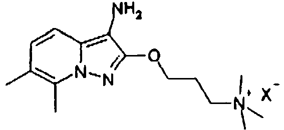 |
| Sel de [3-(3-Amino-6,7-dimethyl-pyrazolo[1,5-a]pyridin-2-yloxy]-propyl]-trimethyl-ammonium |
| 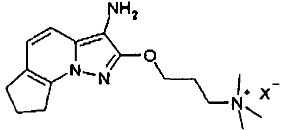 |
| Sel de [3-(3-Amino-7,8-dihydro-6H-cyclopenta[e]pyrazolo[1,5-a]pyridin-2-yloxy)-propyl]-trimethyl-ammonium |
| 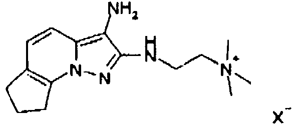 |
| Sel de {2-[(3-amino-7,8-dihydro-6H-cyclopenta[e]pyrazolo[1,5-a]pyridin-2-yl)amino]-ethyl}-trimethyl-ammonium |
| 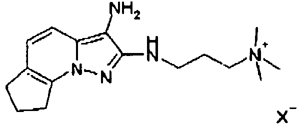 |
| Sel de {3-[(3-amino-7,8-dihydro-6H-cyclopenta[e]pyrazolo[1,5-a]pyridin-2-yl)amino]-propyl}-trimethyl-ammonlum |
| 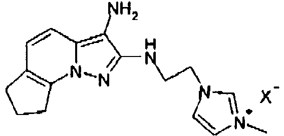 |
| Sel de 1-{2-[(3-amino-7,8-dihydro-6H-oyclopenta[e]pyrazolo[1,5-a]pyridin-2-yl)amino]ethyl}-3-methyl-1 H-imidazol-3-ium |
| 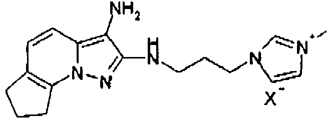 |
| Sel de 1-(3-[(3-amino-7,8-dihydro-6H-cyclopenta[e]pyrazolo[1,6-a]pyridin-2-yl)amino]propyl}-3-methyl-1H-imidazol-3-ium |
| 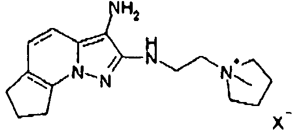 |
| Sel de 1-{2-[(3-amino-7,8-dihydro-8H-cyclopenta[e]pyrazolo[1,5-a]pyridin-2-yl)amino]ethyl]-1-methylpyrrolidinium |
| 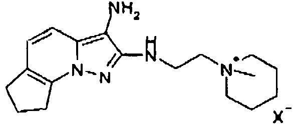 |
| Sel de 1-{2-[(3-amino-7,8-dihydro-6H-cyclopenta[e]pyrazolo[1,5-a]pyridin-2-yl)amino]ethyl}-1-methylpiperidinium |
| 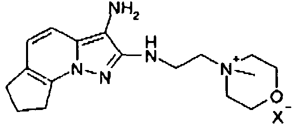 |
| Sel de 4-{2-[(3-amino-7,8-dihydro-6H-cyclopenta[e]pyrazolo[1,5-a]pyridin-2-yl)amino]ethyl}-4-methylmorpholin-4-ium |
| 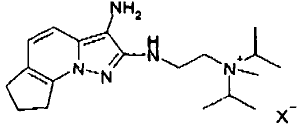 |
| Sel de {2-[(3-amino-7,8-dihydro-6H-cyclopenta[e]pyrazolo[1,5-a]pyridin-2-yl)amino]ethyl}-diisopropyl-methyl-ammonium |
| 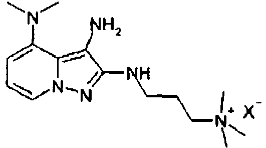 |
| Sel de [3-(3-Amino-4-dimethylamino-pyrazolo[1,5-a]pyridin-2-ylamino)-propyl]-trimethyl-ammonium |
| 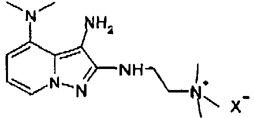 |
| Sel de [2-(3-Amino-4-dimethylamino-pyrazolo[1,5-a]pyridin-2-ylamino)-ethyl]-trimethyl-ammonium |
| 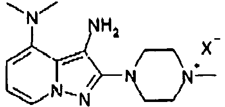 |
| Sel de 4-(3-Amino-4-dimethylamino-pyrazolo[1,5-a]pyridin-2-yl)-1-methyl-piperazin-1-ium |
| 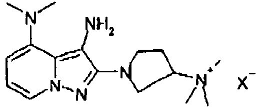 |
| Sel de [1-(3-Amino-4-dimethylamino-pyrazolo[1,5-a]pyridin-2-yl)-pyrrolidin-3-yl]-trimethyl-ammonium |
| 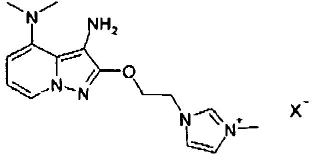 |
| Sel de 3-[2-(3-Amino-4-dimethylamino-pyrazolo[1,5-a]pyridin-2-yloxy)-ethyl]-1-methyl-3H-imidazol-1-ium |
| 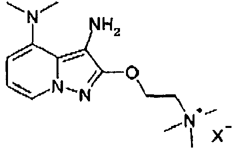 |
| Sel de [2-(3-Amino-4-dimethylamino-pyrazolo[1,5-a]pyridin-2-yloxy)-ethyl]-trimethyl-ammonium |
| 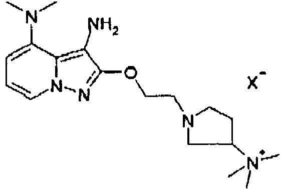 |
| Sel de {1-[2-(3-Amino-4-dimethylamino-pyrazolo[1,5-a]pyridin-2-yloxy)-ethyl]-pyrrolidin-3-yl}-trimethyl-ammonium |
| 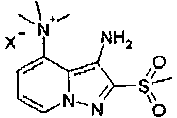 |
| Sel de (3-Amino-2-methanosulfonyl-pyrazolo[1,5-a]pyridin-4-yl)-trimethyl-ammonium |
| 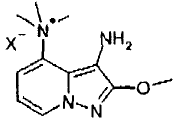 |
| Sel de (3-Amino-2-methoxy-pyrazolo[1,5-a]pyridin-4-yl)-trimethyl-ammonium |

11. Composition selon l'une quelconque des revendications précédentes **caractérisée en ce que** les éthers de cellulose non ioniques sont choisis parmi les alkylcelluloses les hydroxyalkylcelluloses et les hydroxyalkyl-alkylcelluloses.

12. Composition selon la revendication 11 **caractérisée en ce que** les hydroxyalkylcelluloses sont choisies parmi les hydroxyméthylcelluloses, les hydroxyéthylcelluloses et les hydroxypropylcelluloses.

13. Composition selon l'une quelconque des revendications précédentes dans laquelle les celluloses sont des celluloses modifiées par un groupement hydrophobe, ces celluloses pouvant être cationiques ou non cationiques.

14. Composition selon l'une quelconque des revendications précédentes, **caractérisée par le fait que** les dérivés de cellulose sont des celluloses quaternisée comportant au moins une chaîne grasse (C₈-C₃₀) et choisis dans le groupe formé par les celluloses et les hydroxyéthylcelluloses, quaternisées et modifiées par des groupements alkyle, ou arylalkyle, ou alkylaryle comportant de 8 à 30 atomes de carbone, ou des mélanges de ceux-ci.

15. Composition selon l'une quelconque des revendications précédentes comprenant un coupleur choisi parmi les métaphénylènediamines, les méta-aminophénols, les métadiphénols, les coupleurs naphtaléniques, les coupleurs hétérocycliques et leurs sels d'addition.

16. Procédé de teinture d'oxydation des fibres kératiniques, **caractérisé en ce qu'**on applique sur les fibres une composition tinctoriale telle que définie dans l'une quelconque des revendications 1 à 15 en présence d'un agent oxydant pendant un temps suffisant pour développer la coloration désirée.

17. Dispositif à plusieurs compartiments dans lequel un premier compartiment contient une composition tinctoriale telle que définie à l'une quelconque des revendications 1 à 15 et un deuxième compartiment contient un agent oxydant.

## Patentansprüche

1. Färbezusammensetzung, umfassend in einem geeigneten Färbemedium mindestens eine Aminopyrazolopyridin-Oxidationsbase, ausgewählt aus den Basen der Formel (I) oder (II): 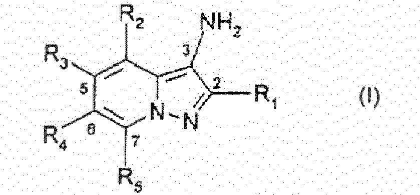 worin:
- R₁, R₂, R₃, R₄ und R₅ gleich oder verschieben sind und für ein Wasserstoff- oder Halogenatom; einen -NHSO₃H-Rest; einen Hydroxylrest; einen (C₁-C₄)-Alkylrest; einer (C₁-C₄)-Alkoxyrest; einem (C₁-C₄)-Alkylthiorest; Mono-(C₁-C₄)-alkylamino; einen Di-(C₁-C₄)-alkylaminorest, worin die beiden Alkyl-gruppen zusammen mit dem Stickstoffatom, an das sie gebunden sind, einen Ring bilden können, der durch ein oder mehrere Stickstoff-, Sauerstoff- oder Schwefelatome unterbrochen sein kann; einen Heterocyclus; einen Nitrorest; einen Phenylrest; einen Carbonylrest; einen (C₁-C₄)-Alkoxycarbonylrest; einen Carboxamidorest; einen Cyanorest; einen Aminorest; einen Sulfonylrest; einen -CO₂H-Rest; einen -SO₃H-Rest, einen -PO₃H₂-Rest; einen -PO₄H₂-Rest oder eine Gruppe: 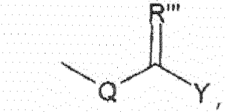 worin R'" für ein Sauerstoff- oder Stickstoffatom steht, Q für ein Sauerstoffatom oder eine NH- oder NH-(C₁-C₄)-Alkylgruppe steht und Y für einen Hydroxyl-, Amino-, C₁-C₄-Alkyl-, (C₁-C₄ -Alkoxy-, (C₁-C₄)-Alkylamino- oder Di-(C₁-C₄)-alkylaminorest steht, stehen; 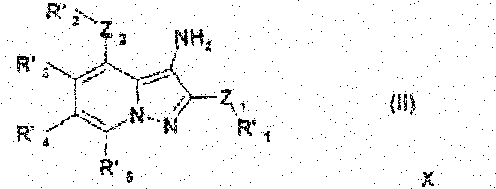
worin
• Z₁ und Z₂ unabhängig voreinander für
- eine kovalente Einfachbindung,
- einen zweiwertigen Rest, ausgewählt aus
- einem -O(CH₂)ₚ-Rest, wobei p für eine ganze Zahl im Bereich von 0 bis 6 steht,
- einem -NR'₆(CH₂)_{q}(C₆H₄)ₜ-Rest, wobei q für eine ganze Zahl im Bereich von 0 bis 6 steht und t für 0 oder 1 steht und R'₆ für ein Wasserstoffatom oder einen C₁-C₆-Alkylrest, der gegebenenfalls durch eine oder mehrere Hydroxygruppen substituiert ist, steht, stehen,
• Z₁ auch für einen zweiwertigen -S-, -SO- oder -SO₂-Rest stehen kann, wenn R'₁ für einen Methylrest steht,
• R'₁ und R'₂ unabhängig voreinander für:
- einen Wasserstoff,
- einen C₁-C₁₀-Alkylrest, der gegebenenfalls substituiert und gegebenenfalls durch ein Heteroatom oder eine Gruppierung, ausgewählt aus O, N, Si, S, SO und SO₂, unterbrochen ist,
- ein Halogen,
- einen SO₃H-Rest,
- einen gesättigten, ungesättigten oder aromatischen, substituierten oder unsubstituierten 5- bis 8-gliedrigen Ring, der gegebenenfalls ein oder mehrere Heteroatome oder Gruppen, ausgewählt aus N, O, S, SO₂ und -CO-, enthält und kationisch sein und/oder durch einen kationischen Rest substituiert sein kann,
- eine -N⁺R₁₇R₁₈R₁₉-Gruppe, wobei R₁₇, R₁₈ und R₁₉ für lineare oder verzweigte C₁-C₅-Alkyl-gruppen, die gegebenenfalls durch eine oder mehrere Hydroxygruppen substituiert sind, stehen,
stehen,
wenn Z₁ bzw. Z₂ für eine kovalente Bindung steht,
R'₁ bzw. R'₂ auch für:
- einen gegebenenfalls substituierten C₁-C₆-Alkylcarbonylrest,
- einen -O-CO-R-, -CO-O-R-, NR-CO-R'- oder -CO-NRR'-Rest, worin R und R' unabhängig voneinander für ein Wasserstoffatom oder einen gegebenenfalls substituierten C₁-C₆-Alkylrest stehen,
stehen können,
• R'₃, R'₄ und R'₅ gleich oder verschieden sind und für
- ein Wasserstoffatom,
- einen Hydroxylrest,
- einen C₁-C₆-Alkoxyrest,
- einen C₁-C₆-Alkylthiorest,
- einen Aminorest,
- einen Monoalkylaminorest,
- einen C₁-C₆-Dialkylaminorest, worin die Alkyl-reste mit dem Stickstoffatom, an das sie gebunden sind, einen gesättigten, ungesättigten, aromarischen oder nichtaromatischen 5-bis 8-gliedrigen Heterocyclus, der gegebenenfalls ein oder mehrere Heteroatome oder Gruppen, ausgewählt aus N, O, S, SO₂ und CO, enthält und kationisch sein und/oder durch einen kationischen Rest substituiert sein kann, bilden können,
- einen gegebenenfalls substituierten C₁-C₆-Alkylcarbonylrest,
- einen -O-CO-R-, -CO-O-R-, NR-CO-R'- oder -CO-NRR'-Rest, wobei R und R' wie oben definiert sind,
- ein Halogen,
- einen -NHSO₃H-Rest,
- einen gegebenenfalls substituierten C₁-C₄-Alkylrest,
- einen gesättigten, ungesättigten oder aromatischen, gegebenenfalls substituierten Kohlenstoffring
stehen,
- wobei R'₃, R'₄ und R'₅ paarweise einen gesättigten oder ungesättigten Ring bilden können,
• X für ein Ion oder eine Gruppe von Ionen steht, so dass die Elektronegativität der Verbindung der Formel (II) gewährleistet ist,
mit der Maßgabe, dass mindestens eine der Gruppen R'₁ und R'₂ für einen kationischen Rest steht,
• einen oder mehrere Kuppler,
• ein oder mehrere Assoziativpolymere, die aus auf Cellulose basierenden Assoziativpolymeren ausgewählt sind.

2. Zusammensetzung nach Anspruch 1, **dadurch gekennzeichnet, dass** die Verwindungen der Formel (I) aus den Verwindungen der folgenden Unterformel und deren Additionssalzen mit einer Säure oder mit einer Base ausgewählt sind: 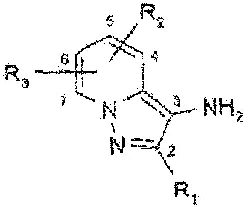 worin:
- R₁, R₂ und R₃ gleich oder verschieden sind und für ein Wasserstoff- oder Halogenatom; einen Hydroxylrest; einen (C₁-C₄)-Alkylrest; einen (C₁-C₄)-Alkylthiorest, einen (C₁-C₄)-Alkoxyrest; einen -NHSO₃H-Rest; einen Aminorest; einen (C₁-C₄)-Alkylaminorest; einen Di-(C₁-C₄)-alkylaminorest, worin die beiden Alkylgruppen zusammen mit dem Stickstoffatom, an das sie gebunden sind, einen Ring bilden können, der durch ein oder mehrere Stickstoff-, Sauerstoff- oder Schwefelatome unterbrochen sein kann, einen Heterocyclus; einen Sulfonamidrest; einen Carbonylrest; einem C₁-C₄)-Alkoxycarbonylrest; einen Carboxamidorest oder eine Gruppe der folgenden Formel: 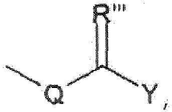 worin R'" für ein Sauerstoff- oder Stickstoffatom steht, X für ein Sauerstoffatom oder eine NH- oder NH-(C₁-C₄)-Alkylgruppe steht und Y für einen Hydroxyl-, Amino-, C₁-C₄-Alkyl-, (C₁-C₄)-Alkoxy-, (C₁-C₄)-Alkylamino- oder Di-(C₁-C₄)-alkylaminorest steht, stehen.

3. Zusammensetzung nach Anspruch 1, **dadurch gekennzeichnet, dass** die 3-Aminopyrazolo[1,5-a]pyridine der Formel (I) ausgewählt sind aus:
- Pyrazolo[1,5-a]pyridin-3-ylamin;
- 2-Acetylaminopyrazolo-[1,5-a]pyridin-3-ylamin;
- 2-Morpholin-4-yl-pyrazol[1,5-a]pyridin-3-yl-amin;
- 3-Aminopyrazolo[1,5-a]pyridin-2-carbonsäure;
- 2-Methoxypyrazolo[1,5-a]pyridin-3-ylamino;
- (3-Aminopyrazolo[1,5-a]pyridin-7-yl)methanol;
- 2-3-Aminopyrazolo[1,5-a]pyridin-5-yl)ethanol;
- 2-(3-Aminopyrazolo[1,5-a]pyridin-7-yl)ethanol;
- (3-Aminopyrazolo[1,5-a]pyridin-2-yl)ethanol;
- 3,6-Diaminopyrazolo[1,5-a]pyridin;
- 3,4-Diaminopyrazolo[1,5-a]pyridin;
- Pyrazol[1,5-a]pyridin-3,7-diamin;
- 7-Morpholin-4-yl-pyrazolo[1,5-a]pyridin-3-yl-amin;
- Pyrazolo[1,5-a]pyridin-3,5-diamin;
- 5-Morpholin-4-yl-pyrazolo[1,5-a]pyridin-3-yl-amin;
- 2-[(3-Aminopyrazolo[1,5-a]pyridin-5-yl)(2-hydroxyethyl)amino]ethanol;
- 2-[(3-Aminopyrazolo[1,5-a]pyridin-7-yl)(2-hydroxyethyl)amino]ethanol;
- 3-Aminopyrazolo[1,5-a]pyridin-5-ol;
- 3-Aminopyrazolo[1,5-a]pyridin-4-ol;
- 3-Aminopyrazolo[1,5-a]pyridin-6-ol;
- 3-Aminopyrazolo[1,5-a]pyridin-7-ol;
und deren Additionssalzen mit einer Säure oder mit einer Base.

4. Zusammensetzung nach Anspruch 1, **dadurch gekennzeichnet, dass** in der Formel (II) Z₁ und/oder Z₂ für eine kovalente Bildung, einen -NR'₆(CH₂)_{q}-Rest oder einen -O(CH₂)ₚ-Rest stehen und R'₁ und/oder R'₂ für einen kationischen Rest stehen.

5. Zusammensetzung nach Anspruch 4, worin R'₁ oder R'₂ für Imidazolgruppen, die durch einen quaternären Ammoniumrest substituiert sind, oder Imidazoliumgruppen, Piperazingruppen, die durch einen quaternären Ammoniumrest substituiert sind, oder Piperaziniumgruppen, Pyrrolidingruppen, die durch einen quaternären Ammoniumrest substituiert sind, oder Pyrrolidiniumgruppen oder Diazepangruppen, die durch einen quaternären Ammoniumrest substituiert sind, oder Diazepaniumgruppen stehen.

6. Zusammensetzung nach Anspruch 4, worin R'₁ und R'₂ unabhängig voreinander für ein Wasserstoffatom oder eine Trialkylammonium-, Tri(hydroxyalkyl)-ammonium-, (Hydroxyalkyl)dialkylammonium- oder Di(hydroxyalkyl)alkylammoniumgruppe stehen.

7. Zusammensetzung nach einem der vorhergehenden Ansprüche, worin die Reste R'₃, R'₄ und R'₅ der Formel (II) unabhängig voreinander für ein Wasserstoffatom oder einen gegebenenfalls substituierten C₁-C₄-Alkylrest stehen.

8. Zusammensetzung nach einem der Ansprüche 1 bis 5,
worin die Verbindung der Formel (II) 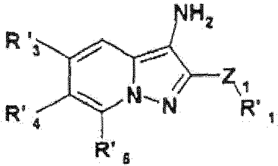 entspricht, worin Z₁, R'₁, R'₃, R'₄ und R'₅ wie in einem der vorhergehenden Ansprüche definiert sind.

9. Zusammensetzung nach Anspruch 8, worin Z₁ für eine kovalente Bindung, einen -NR'₆CH₂)_{q}-Rest oder einem -O(CH₂)ₚ-Rest steht und R'₁ für einen kationischen. Rest steht.

10. Zusammensetzung nach einem der vorhergehenden Ansprüche, worin die Verbindung der Formel (II) ausgewählt ist aus:
| |
|---|
| 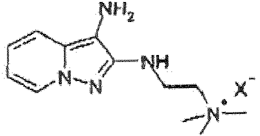 |
| [2-(3-Aminopyrazolo[1,5-a]pyridin-2-ylamino)ethyl]trimethylammoniumsalz |
| 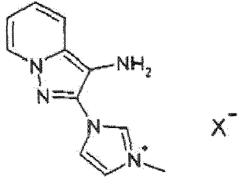 |
| 3-(3-Aminopyrazolo[1,5-a]pyridin-2-yl)-2-methyl-3H-imidazol-1-iumsalz |
| 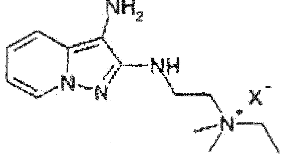 |
| [2-(3-Aminopyrazolo[1,5-a]pyridin-2-ylamino)ethyl]ethyldimethylammoniumsalz |
| 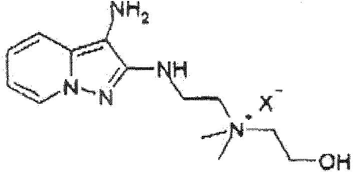 |
| [2-(3-Aminopyrazolo[1,5-a]pyridin-2-ylamino)ethyl](2-hydroxyethyl)dimethylammoniumsalz |
| 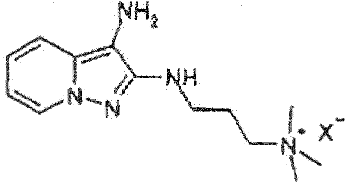 |
| [3-(3-Aminopyrazolo[1,5-a]pyridin-2-ylamino)propyl]trimethylammoniumsalz |
| 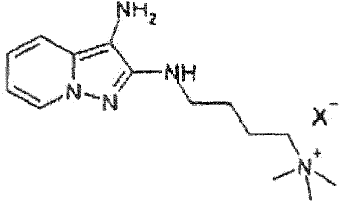 |
| [4-(3-Aminopyrazolo[1,5-a]pyridin-2-ylamino)butyl]-trimethylammoniumsalz |
| 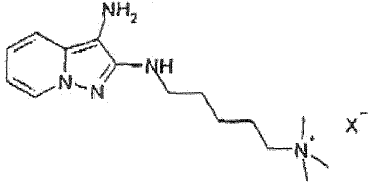 |
| [5-3-Aminopyrazolo[1,5-a]pyridin-2-ylamino)pentyl]trimethylammoniumsalz |
| 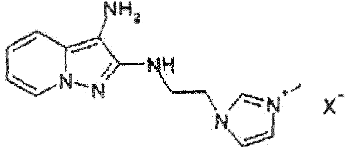 |
| 3-[2-(3-Aminopyrazolo[1,5-a]pyridin-2-ylamino)ethyl]-1-methyl-3H-imidazol-1-iumsalz |
| 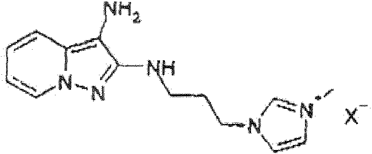 |
| 3-[3-(3-Aminopyrazolo[1,5-a]pyridin-2-ylamino)propyl]-1-methyl-3H-imidazol-1-iumsalz |
| 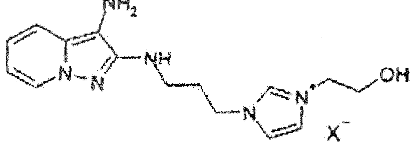 |
| 3-[3-(3-Aminopyrazolo[1,5-a]pyridin-2-ylamino)propyl]-1-(2-hydroxyethyl)-3H-imidazol-1-iumsalz |
| 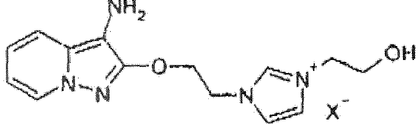 |
| 3-[2-(3-Aminopyrazolo[1,5-a]pyridin-2-yloxy)ethyl]-1-(2-hydroxyethyl)-3H-imidazol-1-iumsalz |
| 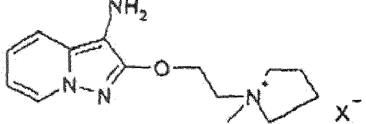 |
| 1-{2-[(3-Aminopyrazolo[1,5-a]pyridin-2-yl)oxy]ethyl}-1-methylpiperidiniumsalz |
| 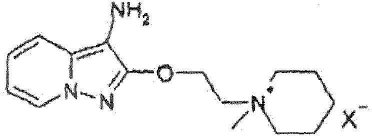 |
| 1-{2-[(3-Aminopyrazolo[1,5-a]pyridin-2-yl)oxylethyl}-1-methylpiperidiniumsalz |
| 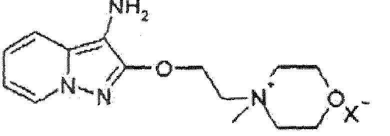 |
| 4-{2-[(3-Aminopyrazolo[1,5-a]pyridin-2-yl)oxy]ethyl}-4-methylmorpholin-4-iumsalz |
| 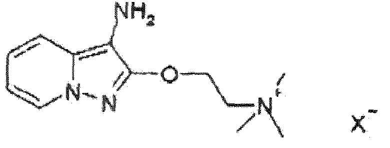 |
| {2-[(3-Aminopyrazolo[1,5-a]pyridin-2-yl)oxy]ethyl}trimethylammoniumsalz |
| 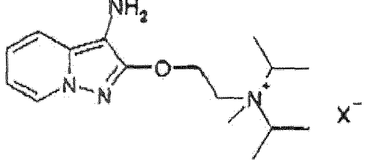 |
| {2-[(3-Aminopyrazolo[1,5-a]pyridin-2-yl)oxy]ethyl}diisopropylmethylammoniumsalz |
| 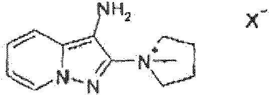 |
| 1-(3-Aminopyrazolo[1,5-a]pyridin-2-yl)-1-methylpyrrolidiniumsalz |
| 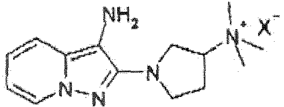 |
| [1-(3-Aminopyrazolo[1,5-a]pyridin-2-yl)pyrrolidin-3-yl]trimethylammoniumsalz |
| 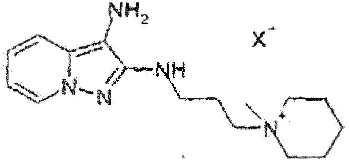 |
| 1-[3-(3-Aminopyrazolo[1,5-a]pyridin-2-ylamino)propyl]-1-methylpiperidiniumsalz |
| 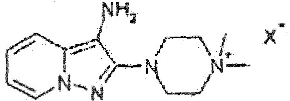 |
| 4-(3-Aminopyrazolo[1,5-a]pyridin-2-yl)-1,1-dimethylpiperazin-1-iumsalz |
| 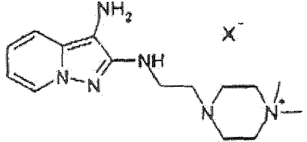 |
| 4-[2-(3-Aminopyrazolo[1,5-a]pyridin-2-ylamino)ethyl]-1,1-dimethylpiperazin-1-iumsalz |
| 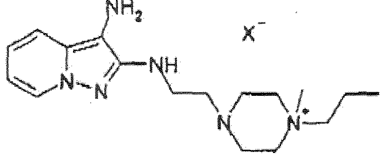 |
| 4-[2-(3-Aminopyrazolo[1,5-a]pyridin-2-ylamino)ethyl]-1-methyl-1-propylpiperazin-1-iumsalz |
| 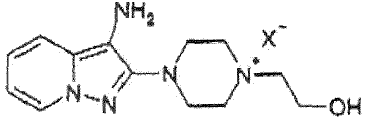 |
| 4-(3-Aminopyrazolo[1,5-a]pyridin-2-yl)-1-(2-hydroxyethyl)piperazin-1-iumsalz |
| 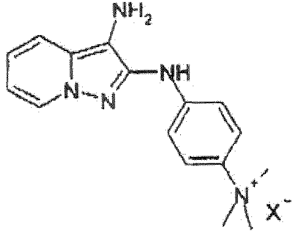 |
| [4-(3-Aminopyrazolo[1,5-a]pyridin-2-ylamino)phenyl]trimethylammoniumsalz |
| 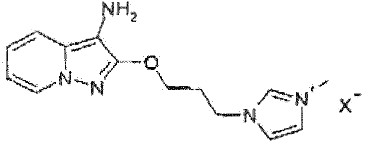 |
| 3-[3-(3-Aminopyrazolo[1,5-a]pyridin-2-yloxy)propyl]-1-methyl-3H-imidazol-1-iumsalz |
| 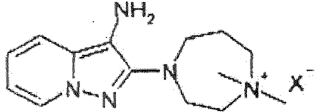 |
| 4-(3-Aminopyrazolo[1,5-a]pyridin-2-yl)-1,1-dimethyl-[1,4]diazepan-1-iumsalz |
| 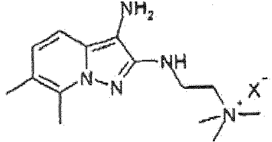 |
| [2-(3-Amino-6,7-dimethylpyrazolo[1,5-a]pyridin-2-ylamino)ethyl]trimethylammoniumsalz |
| 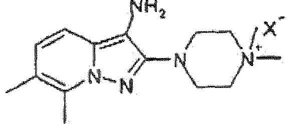 |
| 4-(3-Amino-6,7-dimethylpyrazolo[1,5-a]pyridin-2-yl)-1,1-dimethylpiperazin-1-iumsalz |
| 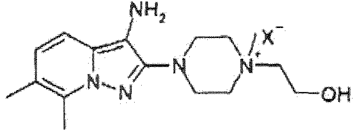 |
| 4-(3-Amino-6,7-dimethylpyrazolo[1,5-a]pyridin-2-yl)-1-(2-hydroxyethyl-1-methylpiperazin-1-iumsalz |
| 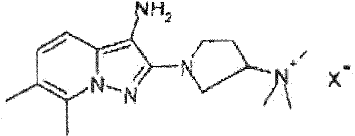 |
| [1-(3-Amino-6,7-dimethylpyrazolo[1,5-a]pyridin-2-yl)pyrrolidin-3-yl]trimethylammoniumsalz |
| 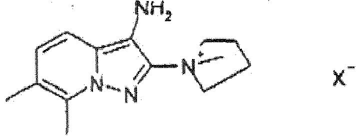 |
| 1-(3-Amino-6,7-dimethylpyrazolo[1,5-a]pyridin-2-yl)-1-methylpyrrolidiniumsalz |
| 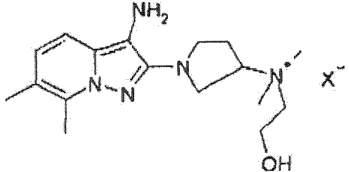 |
| [1-(3-Amino-6,7-dimethylpyrazolo[1,5-a]pyridin-2-yl)pyrrolidin-3-yl](2-hydroxyethyl)dimethylammoniumsalz |
| 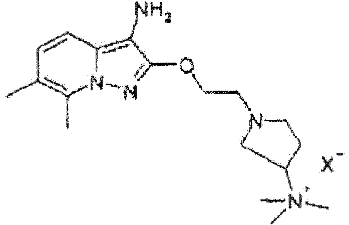 |
| {1-[2-(3-Amino-6,7-dimethylpyrazolo[1,5-a]pyridin-2-yloxy)ethyl]pyrrolidin-3-yl}trimethylammoniumsalz |
| 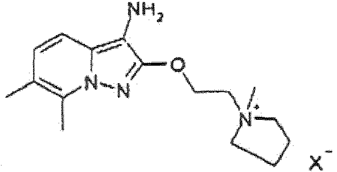 |
| 1-{2-[(3-Amino-6,7-dimethylpyrazolo[1,5-a]pyridin-2-yl)oxy]ethyl}-1-methylpyrrolidiniumsalz |
| 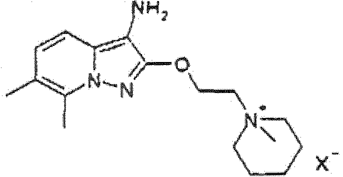 |
| 1-{2-[(3-Amino-6,7-dimethylpyrazolo[1,5-a]pyridin-2-yl)oxy]ethyl}-1-methylpiperidiniumsalz |
| 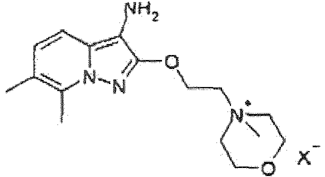 |
| 4-{2-[(3-Amino-6,7-dimethylpyrazolo[1,5-a]pyridin-2-yl)oxy]ethyl}-4-methylmorpholin-4-iumsalz |
| 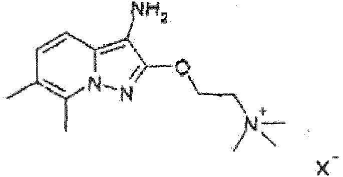 |
| {2-[(3-Amino-6,7-dimethylpyrazolo[1,5-a]pyridin-2-yl)oxy]ethyl}trimethylammoniumsalz |
| 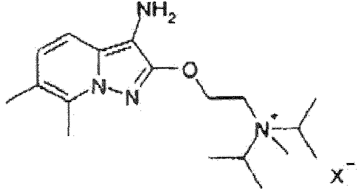 |
| {2-[(3-Amino-6,7-dimethylpyrazolo[1,5-a]pyridin-2-yl)oxy]ethyl}diisopropylmethylammoniumsalz |
| 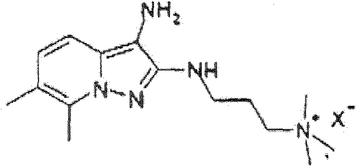 |
| [3-(3-Amino-6,7-dimethylpyrazolo[1,5-a]pyridin-2-ylamino)propyl]trimethylammoniumsalz |
| 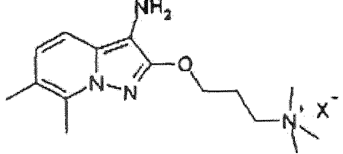 |
| [3-(3-Amino-6,7-dimethylpyrazolo[1,5-a]pyridin-2-yloxy)propyl]trimethylammoniumsalz |
| 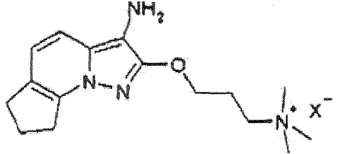 |
| [3-(3-Amino-7,8-dihydro-6H-cyclopenta[e]pyrazolo[1,5-a]pyridin-2-yloxy)propyl]trimethylammoniumsalz |
| 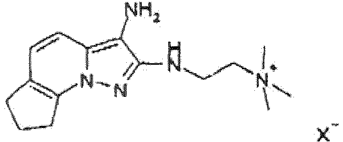 |
| {2-[(3-Amino-7,8-dihydro-6H-cyclopenta[e]pyrazolo[1,5-a]pyridin-2-yl)amino]ethyl}trimethylammoniumsalz |
| 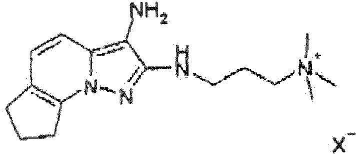 |
| {3-[(3-Amino-7,8-dihydro-6H-cyclopenta[e]pyrazolo[1,5-a]pyridin-2-yl)amino]propyl)trimethylammoniumsalz |
| 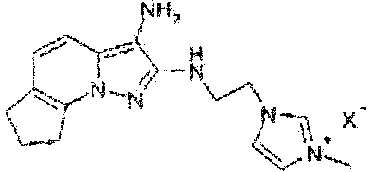 |
| 1-{2-[(3-Amino-7,8-dihydro-6H-cyclopenta[e]pyrazolo[1,5-a]pyridin-2-yl)amino]ethyl}-3-methyl-1H-imidazol-3-iumsalz |
| 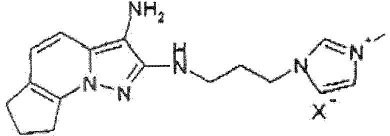 |
| 1-{3-[(3-Amino-7,8-dihydro-6H-cyclopenta[e]pyrazolo[1,5-a]pyridin-2-yl)amino]propyl}-3-methyl-1H-imidazol-3-iumsalz |
| 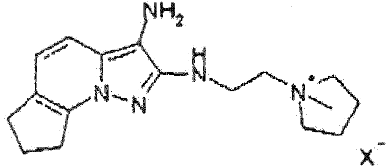 |
| 1-{2-[(3-Amino-7,8-dihydro-6H-cyclopenta[e]pyrazolo[1,5-a]pyridin-2-yl)amino]ethyl}-1-methylpyrrolidiniumsalz |
| 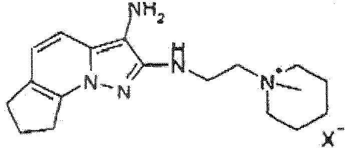 |
| 1-{2-[(3-Amino-7',8-dihydro-6H-cyclopenta[e]pyrazolo[1,5-a]pyridin-2-yl)amino]ethyl}-1-methylpiperidiniumsalz |
| 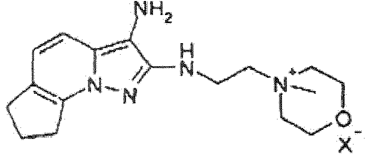 |
| 4-{2-[(3-Amino-7,8-dihydro-6H-cyclopenta[e]pyrazol[1,5-a]pyridin-2-yl)amino]ethyl}-4-methylmorpholin-4-iumsalz |
| 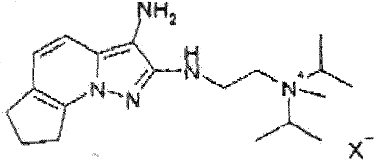 |
| {2-[(3-Amino-7,8-dihydro-6H-cyclopenta[e]pyrazolo[1,5-a]pyridin-2-yl)amino]ethyl}diisopropylmethylammoniumsalz |
| 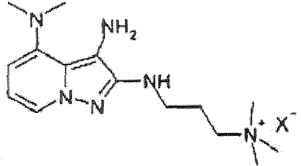 |
| [3-(3-Amino-4-dimethylaminopyrazolo[1,5-a]pyridin-2-ylamino)propyl]trimethylammoniumsalz |
| 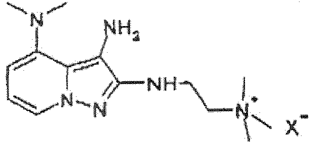 |
| [2-(3-Amino-4-dimethylaminopyrazolo[1,5-a]pyridin-2-ylamino)ethyl]trimethylammoniumsalz |
| 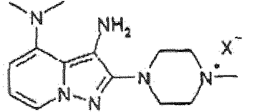 |
| 4-(3-Amino-4-dimethylaminopyrazolo[1,5-a]pyridin-2-yl)-1-methylpiperazin-1-iumsalz |
| 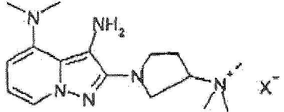 |
| [1-(3-Amino-4-dimethylaminopyrazolo[1,5-a]pyridin-2-yl)pyrrolidin-3-yl]trimethylammoniumsalz |
| 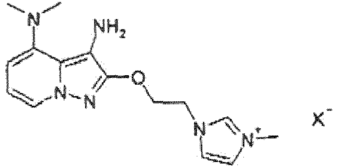 |
| 3-[2-(3-Amino-4-dimethylaminopyrazolo[1,5-a]pyridin-2-yloxy)ethyl]-1-methyl-3H-imidazol-1-iumsalz |
| 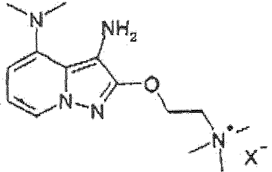 |
| [2-(3-Amino-4-dimethylaminopyrazolo[1,5-a]pyridin-2-yloxy)ethyl]trimethylammoniumsalz |
| 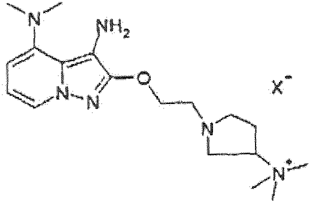 |
| {1-[2-(3-Amino-4-dimethylaminopyrazolo[1,5-a]pyridin-2-yloxy)ethyl]pyrrolidin-3-yl}trimethylammoniumsalz |
| 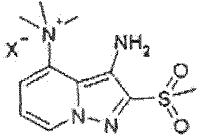 |
| (3-Amino-2-methansulfonylpyrazolo[1,5-a]pyridin-4-yl)-trimethylammoniumsalz |
| 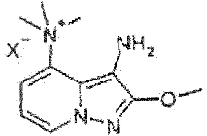 |
| (3-Amino-2-methoxypyrazolo[1,5-a]pyridin-4-yl)trimethylammoniumsalz |

11. Zusammensetzung nach einem der vorhergehenden Ansprüche, dadurch gekenntzeichnet, dass die nichtionischen Celluloseether aus Alkylcellulosen, Hydroxyalkylcellulosen und Hydroxyalkylalkyl-cellulosen ausgewählt sind.

12. Zusammensetzung nach Anspruch 11, **dadurch gekennzeichnet, dass** die Hydroxyalkylcellulosen aus Hydroxymethylcellulosen, Hydroxyethylcellulosen und Hydroxypropylcellulosen ausgewählt sind.

13. Zusammensetzung nach einem der vorhergehenden Anspruche, wobei es sich bei, den Cellulosen um mit einer hydrophoben Gruppe modifizierte Cellulosen handelt, wobei diese Cellulosen kationisch oder nicht kationisch sein können.

14. Zusammensetzung nach einem der vorhergehenden Ansprüche, dadurch gekenntzeichnet, dass es sich bei den Cellulosederivaten um quaternisierte Cellulosen mit mindestens einer (C₈-C₃₀)-Fettkette handelt, die aus der Gruppe bestehend aus Cellulosen und Hydroxyethylcellulosen, die mit Alkyl- oder Arylalkyl- oder Alkylarylgruppen mit 8 bis 30 Kohlenstoffatomen quaternisiert und modifiziert sind, oder Gemischen davon ausgewählt sind.

15. Zusammensetzung nach einem der vorhergehenden Ansprüche, umfassend einen Kuppler, der aus meta-Phenylendiaminen, meta-Aminophenolen, meta-Diphenylen, Naphthalin-Kupplern, heterocyclischen Kupplern und Additionssalzen davon ausgewählt ist.

16. Verfahren zum oxidationsfärben von Keratinfasern, **dadurch gekennzeichnet, dass** man auf die Fasern eine Färbezusammensetzung gemäß einem der Ansprüche 1 bis 15 in Gegenwart eines Oxidationsmittels über einen zur Entwicklung der gewünschten Färbung ausreichenden Zeitraum aufbringt.

17. Vorrichtung mit mehreren Kompartimenten, in der ein erstes Kompartiment eine Färbezusammensetzung gemäß einem der Ansprüche 1 bis 15 enthält und ein zweites Kompartiment ein Oxidationsmittel enthält.

## Claims

1. Dyeing composition comprising, in an appropriate dyeing medium, at least one aminopyrazolopyridine oxidation base chosen from the bases of formula (I) or (II), 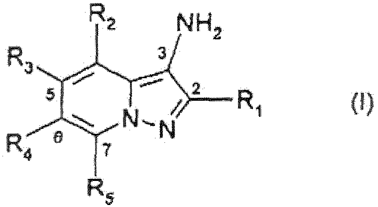 in which:
- R₁, R₂, R₃, R₄ and R₅, which may be identical or different, represent a hydrogen or halogen atom; an -NHSO₃H radical; a hydroxy radical; a (C₁-C₄)alkyl radical; a (C₁-C₄)alkoxy radical; a (C₁-C₄)alkylthio radical; a mono(C₁-C₄)alkylamino; a di(C₁-C₄)alkylamino radical in which the two alkyl groups can, together with the nitrogen atom to which they are bonded, form a ring which may be interrupted with one or more nitrogen, oxygen or sulfur atoms; a heterocycle; a nitro radical; a phenol radical; a carboxyl radical; a (C₁-C₄)alkoxycarbonyl radical; a carboxamido radical; a cyano radical; an amino radical; a sulfonyl radical; a -CO₂H radical; an -SO₃H radical; a -PO₃H₂ radical; a -PO₄H₂ radical; or a group: 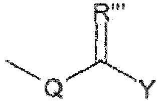 in which R"' represents an oxygen or nitrogen atom, Q represents an oxygen atom or an NH or NH(C₁-C₄)allkyl group, and Y represents a hydroxyl, amino, (C₁-C₄)alkyl, (C₁-C₄)alkoxy, (C₁-C₄)alkylamino or di(C₁-C₄)alkylamino radical, 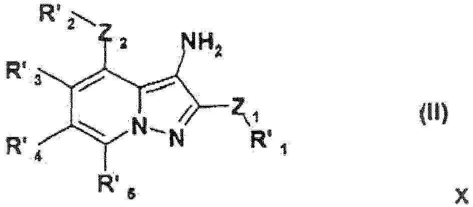
in which
• Z₁ Z₂ independently represent:
- a sample covalent bond,
- a divalent radical chosen from:
- an -O(CH₂)ₚ- radical, p demoting an integer ranting from 0 to 6,
- an -NR'₆(CH₂)_{q}(C₆H₄)ₜ- radical, q demoting an integer ranging from 0 to 6 and t demoting 0 or 1, and R'₆ representing a hydrogen atom or a C₁-C₆ alkyl radial optionally substituted with one or more hydroxyl groups,
• Z₁ can also represent a bivalent radical -S-, -SO-or -SO₂- when R'₁ is a methyl radical,
• R'₁ and R'₂ independently represent:
- a hydrogen,
- an optionally substitutes C₁-C₁₀ alkyl radical optionally interrupted with a heteroatom or a group chosen from O, N, Si, S, SO and SO₂,
- a halogen,
- an SO₃H radical,
- a saturated, unsaturated or aromatic and substituted or unsubstituted 5- to 8-membered ring optionally comprising one or more heteroatoms or groups chosen from N, O, S, SO₂ and -CO-, it being possible for the ring to be cationic and/or substituted with a cationic radical,
- an -N⁺R₁₇R₁₈R₁₉ group, R₁₇, R₁₈ and R₁₉ being linear or branched C₁-C₅ alkyls which are optionally substituted with one or more hydroxyl groups,
when Z₁, respectively Z₂, represents a covalent bond, then R'₁, respectively R'₂, can also represent:
- an optionally substituted C₁-C₆ alkylcarbonyl radical,
- an -O-CO-R, -CO-O-R, NR-CO-R' or -CO-NRR' radical in which R and R' independently represent a hydrogen atom or an optionally substituted C₁-C₆ alkyl radical,
• R'₃, R'₄ and R'₅, which may be identical or different, represent:
- a hydrogen atom,
- a hydroxyl radical,
- a C₁-C₆ alkoxy radical,
- a C₁-C₆ alkylthio radical,
- an amino radical,
- a monoalkylamino radical,
- a C₁-C₆ dialkylamino radical in which the alkyl radicals can form, with the nitrogen atom to which they are attached, a saturated or unsaturated and aromatic or non-aromatic 5- to 8-membered heterocycle which can comprise one or more heteroatoms or groups chosen from N, O, S, SO₂ and CO, it bering possible for the heterocycle to be cationic and/or substituted with a cationic radical,
- an optionally substituted C₁-C₆ alkylcarbonyl radical,
- an -O-CO-R, -CO-O-R, NR-CO-R' or -CO-NRR' radical with R and R' as defined above,
- a halogen,
- an -NHSO₃H radical,
- an optionally substituted C₁-C₄ alkyl radical,
- a saturated, unsaturated or aromatic and optionally substituted carbon ring,
- it being possible for R'₃, R'₄ and R'₅ to form, in pairs, a ring which may or may not be saturated,
• X represents an ion or group of ions making it possible to provide the electronegativity of the derivative of formula (II),
with the condition that at least one of the R'₁ and R'₂ groups represents a cationic radical,
• one or more couplers,
• one or more associative polymers chosen from cellulose-based associative polymers.

2. Composition according to Claim 1, **characterized in that** the compounds of formula (I) are chosen from the compounds of subformula below, and the addition salts thereof with an acid or with a base: 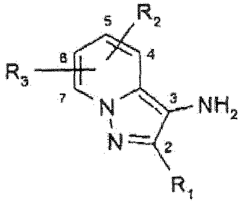 in which:
R₁, R₂ and R₃, which may be identical or different, represent a hydrogen or halogen atom; a hydroxyl radical; a (C₁-C₄)alkyl radical; a (C₁-C₄)alkylthio radical; a (C₁-C₄)alkoxy radical; an -NHSO₃H radical; an amino radical; a (C₁-C₄)alkylamino radical; a di(C₁-C₄)alkylamino radical in which the two alkyl groups can, together with the nitrogen atom to which they are bonded, form a ring which may be interrupted with one or more nitrogen, oxygen or sulfur atoms; a heterocycle; a sulfonamide radical; a carbonyl radical; a (C₁-C₄)alkoxycarbonyl radical; a carboxamido radical; or a group of formula bellow: 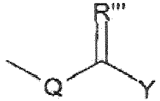 in which R"' represents an oxygen or nitrogen atom, X represents an oxygen atom or an NH or NH(C₁-C₄)alkyl group, and Y represents a hydroxyl, amino, (C₁-C₄)alkyl, (C₁-C₄)alkoxy, (C₁-C₄)alkylamino or di(C₁-C₄)alkylamino radical.

3. Composition according to Claim 1, **characterized in that** the 3-aminopyrazolo[1,5-a]pyridines of formula (I) are chosen from:
- pyrazolo[1,5-a]pyridin-3-ylamine;
- 2-acetylaminopyrazolo[1,5-a]pyridin-3-ylamine;
- 2-morpholin-4-ylpyrazol[1,5-a]pyridin-3-ylamine;
- 3-aminopyrazolo[1,5-a]pyridine-2-carboxylic acid;
- 2-methoxypyrazolo[1,5-a]pyridin-3-ylamine;
- (3-aminopyrazolo[1,5-a]pyridin-7-yl)methanol;
- 2-(3-aminopyrazolo[1,5-a]pyridin-5-yl)ethanol;
- 2-(3-aminopyrazolo[1,5-a]pyridin-7-yl)ethanol;
- (3-aminopyrazolo[1,5-a]pyridin-2-yl)methanol;
- 3,6-diaminopyrazolo[1,5-a]pyridine;
- 3,4-diaminopyrazolo[1,5-a]pyridine;
- pyrazolo[1,5-a]pyridine-3,7-diamine;
- 7-morpholin-4-ylpyrazolo[1,5-a]pyridin-3-ylamine;
- pyrazolo[1,5-a]pyridine-3,5-diamine;
- 5-morpholin-4-ylpyrazolo[1,5-a]pyridin-3-ylamine;
- 2-[(3-aminopyrazolo[1,5-a]pyridin-5-yl)(2-hydroxy-ethyl)amino]ethanol;
- 2-[(3-aminopyrazolo[1,5-a]pyridin-7-yl)(2-hydroxy-ethyl)amino]ethanol;
- 3-aminopyrazolo[1,5-a]pyridin-5-ol;
- 3-aminopyrazolo[1,5-a]pyridin-4-ol;
- 3-aminopyrazolo[1,5-a]pyridin-6-ol;
- 3-aminopyrazolo[1,5-a]pyridin-7-ol;
and the addition salts thereof with an acid or with a base.

4. Composition according to Claim 1, **characterised in that**, in formula (II), Z₁ and/or Z₂ represents a covalent bond, an -NR'₆(CH₂)_{q}- radical or an -O(CH₂)ₚ-radical and R'₁ and/or R'₂ is a cationic radical.

5. Composition according to Claim 4, in which R'₁ or R'₂ denotes imidazoles substituted with a quaternary ammonium radical or imidazoliums, piperazines substituted with a quaternary ammonium radical or piperaziniums, pyrrolidones substituted with a quaternary ammonium radical or pyrrolidiniums, or diazepanes substituted with a quaternary ammonium radical or diazepaniums.

6. Composition according to Claim 4, in which R'₁ and R'₂ independently represent a hydrogen atom or a trialkylammonium, tri(hydroxyalkyl)ammonium, (hydroxy-alkyl)dialkylammonium or di(hydroxyalkyl)alkylammonium group.

7. Composition according to any one of the preceding claims, in which the R'₃, R'₄ and R'₅ radicals of formule (II) independently represent a hydrogen atom or an optionally substituted C₁-C₄ alkyl radical.

8. Composition according to any one of the receding claims, in which the compound of formula (II) corresponds to: 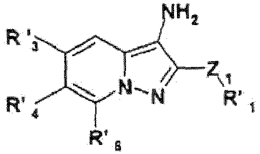 in which Z₁, R'₁, R'₃, R'₄ and R'₅ are as defined in any one of the preceding claims.

9. composition according to Claim 8, in which Z₁ represents a covalent bond, an -NR'₆(CH₂)_{q}- radical or an -O(CH₂)ₚ- radical and R'₁ is a cationic radical.

10. Composition according to any one of the preceding claims, in which the compound of formula (II) is chosen from:
| |
|---|
| 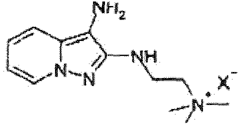 |
| [2-(3-aminopyrazolo[1,5-a]pyridin-2-ylamino)ethyl]trimethylammonium salt |
| 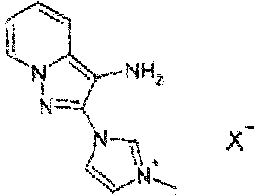 |
| 3-(3-aminopyrazolo[1,5-a]pyridin-2-yl)-1-methyl-3H-imidazol-1-ium salt |
| 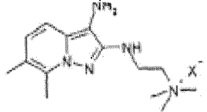 |
| [2-(3-aminopyrazolo[1,5-a]pyridin-2-ylamino)ethyl]ethyldimethylammonium salt |
| 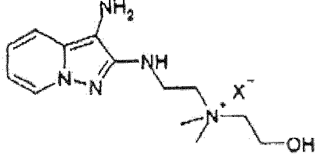 |
| [2-(3-aminopyrazolo[1,5-a]pyridin-2-ylamino)ethyl](2-hydroxyethyl)dimethylammonium salt |
| 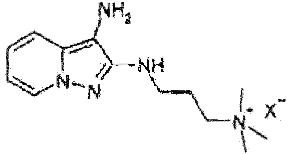 |
| [3-(3-aminopyrazolo[1,5-a]pyridin-2-ylamino)propyl]trimethylammonium salt |
| 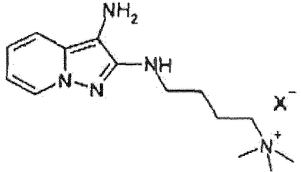 |
| [4-(3-aminopyrazolo[1,5-a]pyridin-2-ylamino)butyl]trimethylammonium salt |
| 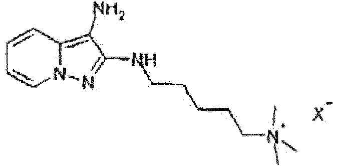 |
| [5-(3-aminopyrazolo[1,5-a]pyridin-2-ylamino)pentyl]trimethylammonium salt |
| 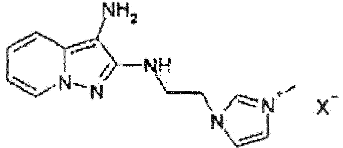 |
| 3-[2-(3-aminopyrazolo[1,5-a]pyridin-2-ylamino)ethyl]-1-methyl-3H-imidazol-1-ium salt |
| 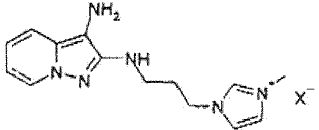 |
| 3-[3-(3-aminopyrazolo[1,5-a]pyridin-2-ylamino)propyl]-1-methyl-3H-imidazol-1-ium salt |
| 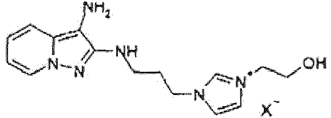 |
| 3-[3-(3-aminopyrazolo[1,5-a]pyridin-2-ylamino)propyl]-1-(2-hydroxyethyl)-3H-imidazol-1-ium salt |
| 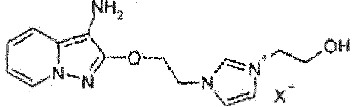 |
| 3-[2-(3-aminopyrazolo[1,5-a]pyridin-2-yloxy)ethyl]-1-(2-hydroxyethyl)-3H-imidazol-1-ium salt |
| 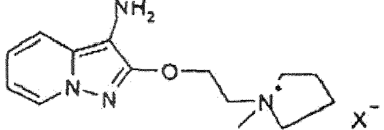 |
| 1-{2-[(3-aminopyrazolo[1,5-a]pyridin-2-yl)oxy]ethyl}-1-methylpyrrolidinium salt |
| 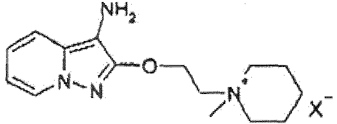 |
| 1-{2-[(3-aminopyrazolo[1,5-a]pyridin-2-yl)oxy]ethyl}-1-methylpiperidinium salt |
| 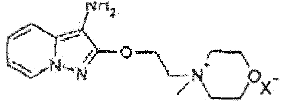 |
| 4-{2-[(3-aminopyrazolo[1,5-a]pyridin-2-yl)oxy]ethyl}-4-methylmorpholin-4-ium salt |
| 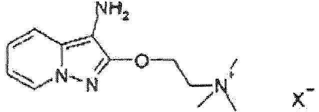 |
| {2-[(3-aminopyrazolo[1,5-a]pyridin-2-yl)oxy]ethyl}trimethylimmonium salt |
| 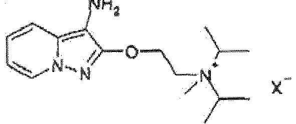 |
| {2-[(3-aminopyrazolo[1,5-a]pyridin-2-yl)oxy]ethy}diisopropylmethylammonium salt |
| 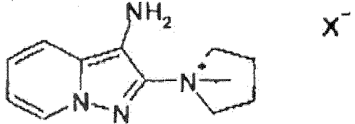 |
| 1-(3-aminopyrazolo[1,5-a]pyridin-2-yl)-1-methylpyrrolidinium salt |
| 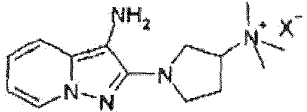 |
| [1-(3-aminopyrazolo[1,5-a-]pyridin-2-yl)pyrrolidin-3-yl]trimethylammonium salt |
| 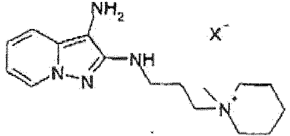 |
| 1-[3-(3-aminopyrazolo[1,5-a]pyridin-2-ylamino)propyl]-1-methylpiperidinium salt |
| 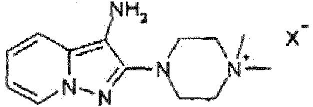 |
| 4-(3-aminopyrazolo[1,5-a]pyridin-2-yl)-1,1-dimethylpiperazin-1-ium salt |
| 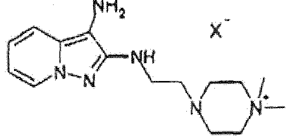 |
| 4-[2-(3-aminopyrazolo[1,5-a]pyridin-2-ylamino)ethyl]-1,1-dimethylpiperazin-1-ium salt |
| 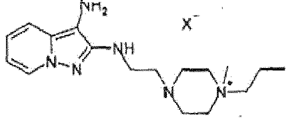 |
| 4[2-(3-aminopyrazolo[1,5-a]pyridin-2-ylamino)ethyl]-1-methyl-1-propylpiperazin-1-ium salt |
| 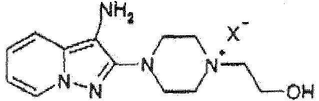 |
| 4-(3-aminopyrazolo[1,5-a]pyridin-2-yl)-1-(2-hydroxyethyl)piperazin-1-ium salt |
| 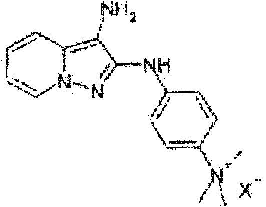 |
| [4-(3-aminopyrazolo[1,5-a]pyridin-2-ylamino)phenyl]trimethylammonium salt |
| 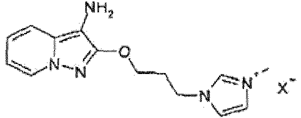 |
| 3-[3-(3-aminopyrazolo[1,5-a]pyridin-2-yloxy)propyl]-1-methyl-3H-imidazol-1-ium salt |
| 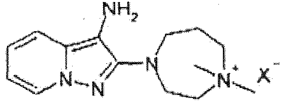 |
| 4-(3-aminopyrazolo[1,5-a]pyridin-2-yl)-1,1-dimethyl-[1,4]diazepan-1-ium salt |
| 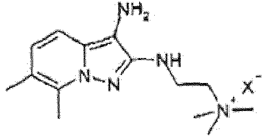 |
| [2-(3-amino-6,7-dimethylpyrazolo[1,5-a-]pyridin-2-ylamino)ethyl]trimethylammonium salt |
| 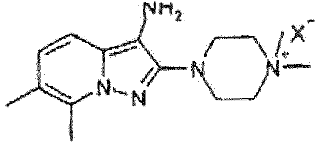 |
| 4-(3-amino-6,7-dimethylpyrazolo[1,5-a]pyridin-2-yl)-1,1-dimethylpiperazin-1-ium salt |
| 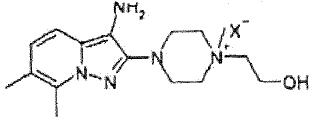 |
| 4-(3-amino-6,7-dimethylpyrazolo[1,5-a]pyridin-2-yl)-1-(2-hydroxyethyl)-1-methylpiperazin-1-ium salt |
| 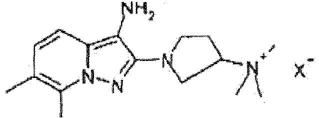 |
| [1-(3-amino-6,7-dimethylpyrazolo[1,5-a]pyridin-2-yl)pyrrolidin-3-yll rimethylammonium salt |
| 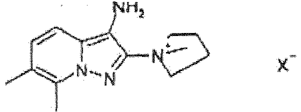 |
| 1-(3-amino-6,7-dimethylpyrazolo[1,5-a]pyridin-2-yl)-1-methylpyrrolidinium salt |
| 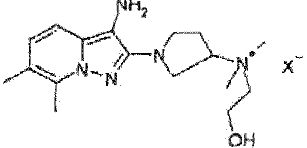 |
| [1-(3-amino-6,7-dimethylpyrazolo[1,5-,a]pyridin-2-yl)pyrrolidin-3-yl](2-hydroxyethyl)dimethylammonium salt |
| 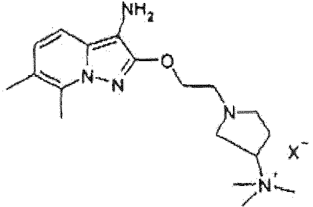 |
| {1-[2-(3-amino-6,7-dimethylpyrazolo[1,5-a]pyridin-2-yloxy)ethyl]pyrrolidin-3-yl}trimethylammonium salt |
| 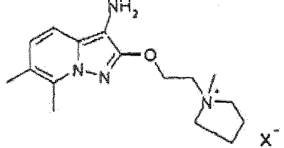 |
| 1-{2-[(3-amino-6,7-dimethylpyrazolo[1,5-a]pyridin-2-y)oxy]ethyl}-1-methylpyrrolidinium salt |
| 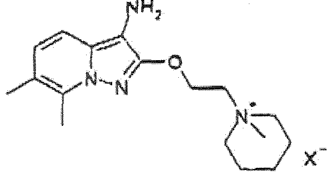 |
| 1-{2-[(3-amino-6,7-dimethylpyrazolo[1,5-a]pyridin-2-yl)oxy]ethyl}-1-methylpiperidinium salt |
| 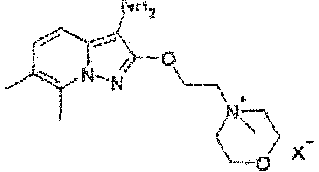 |
| 4-{2-[(3-amino-6,7-dimethylpyrazolo[1,5-a]pyridin-2-yl)oxy]ethyl}-4-methylmorpholin-4-ium salt |
| 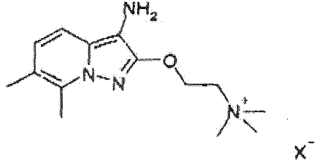 |
| {2-[(3-amino-6,7-dimethylpyrazolo[1,5-a]pyridin-2-yl)oxy]ethyl}trimethylammonium salt |
| 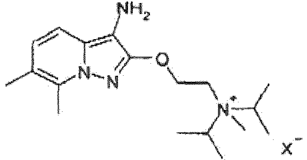 |
| {2-[(3-amino-6,7-dimethylpyrazolo[1,5-a]pyridin-2-yl)oxy]ethyl}diisopropylmethylammonium salt |
| 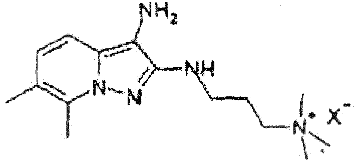 |
| [3-(3-amino-6,7-dimethylpyrazolo[1,5-a]pyridin-2-ylamino)propyl]trimethylammonium salt |
| 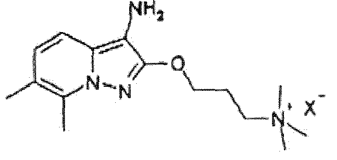 |
| [3-(3-amino-6,7-dimethylpyrazolo[1,5-a]pyridin-2-yloxy)propyl]trimethylammonium salt |
| 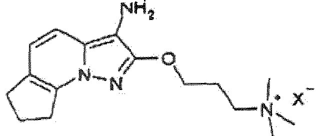 |
| [3-(3-amino-7,8-dihydro-6H-cyclopenta[e]pyrazolo[1,5-a]pyridin-2-yloxy)propyl]trimethylammonium salt |
| 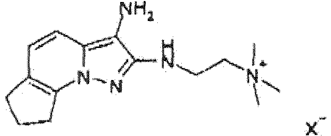 |
| {2-[(3-amino-7,8-dihydro-6H-cyclopenta[e]pyrazolo[1,5-a]pyridin-2-yl)amino]ethyl}trimethylammonium salt |
| 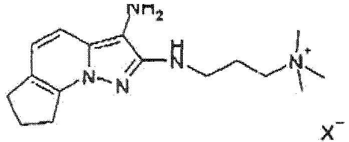 |
| {3-[(3-amino-7,8-dihydro-6H-cyclopenta[e]pyrazolo[1,5-a]pyridin-2-yl)amino]propyl}trimethylammonium salt |
| 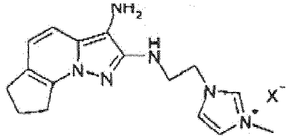 |
| 1-{2-[(3-amino-7,8-dihydro-6H-cyclopenta[e]pyrazolo[1,5-a]pyridin-2-yl)amino]ethyl}-3-methyl-1H-imidazol-3-ium salt |
| 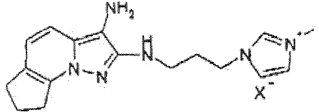 |
| 1-(3-[(3-amino-7,8-dihydro-6H-cyclopenta[e]pyrazolo[1,5-a]pyridin-2-yl)amino]propyl}-3-methyl-1H-imidazol-3-ium salt |
| 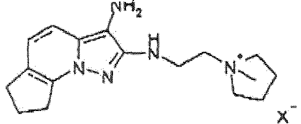 |
| 1-{2-[(3-amino-7,8-dihydro-6Hcyclopenta[e]pyrazolo[1,5-a]pyridin-2-yl)amino]ethyl}-1-methylpyrrolidinium salt |
| 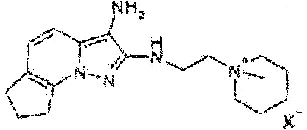 |
| 1-{2-[(3-amino-7,8-dihydro-6H-cyclopenta[e]pyrazolo[1,5-a]pyridin-2-yl)amino]ethyl}-1-methylpiperidinium salt |
| 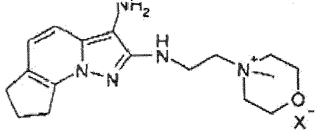 |
| 4-{2-[(3-amino-7,8-dihydro-6H-cyclopenta[e]pyrazolo[1,5-a]pyridin-2-yl)amino]ethyl}4-methylmorpholin-4-ium salt |
| 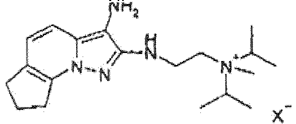 |
| {2-[(3-amino-7,8-dihydro-6H-cyclopenta[e]pyrazolo[1,5-a]pyridin-2-yl)amino]ethyl}diisopropylmethylammonium salt |
| 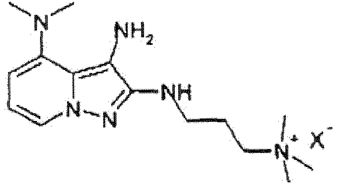 |
| [3-(3-amino-4-dimethylaminopyrazol[1,5-a]pyridin-2-ylamino)propyl]trimethylammonium salt |
| 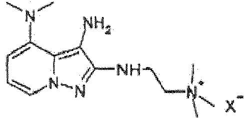 |
| [2-(3-amino-4-dimethylaminopyrazolo[1,5-a]pyridin-2-ylamino)ethyl]trimethylammonium salt |
| 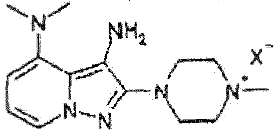 |
| 4-(3-amino-4-dimethylaminopyrazolo[1,5-a]pyridin-2-yl)1-methylpiperazin-1-ium salt |
| 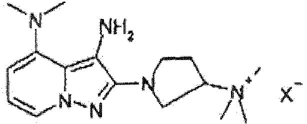 |
| [1-(3-amino-4-dimethylaminopyrazolo[1,5-a]pyridin-2-yl)pyrrolidin-3-yl]trimethylammonium salt |
| 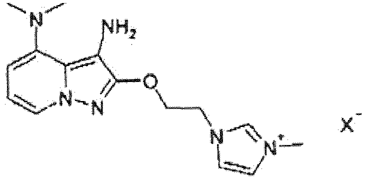 |
| 3-[2-(3-amino-4-dimethylaminopyrazolo[1,5-a]pyridin-2-yloxy)ethyl]-1-methyl-3H-imidazol-1-ium salt |
| 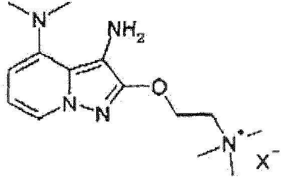 |
| [2-(3-amino-4-dimethylaminopyrazolo[1,5-a]pyridin-2-yloxy)ethyl]trimethylammonium salt |
| 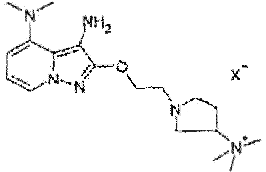 |
| {1-[2-(3-amino-4-dimethylaminopyrazolo[1,5-a]pyridin-2-yloxy)ethyl]pyrrolidin-3-yl}trimethylammonium salt |
| 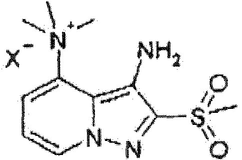 |
| (3-amino-2-methanesulfonylpyrazolo[1,5-a]pyridin-4-yl)-trimethylammonium salt |
| 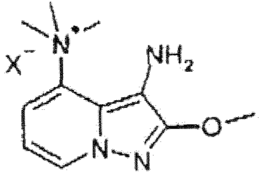 |
| (3-amino-2-methoxypyrazolo[1,5-a]pyridin-4-yl)trimethylammemium salt |

11. Composition according to any one of the preceding claims, **characterized in that** the non-ionic cellulose ethers are chosen from alkylcelluloses, hydroxyalkyl-celluloses and hydroxyalkylalkylcelluloses.

12. Composition according to Claim 11, **characterized in that** the hydroxyalkylcelluloses are chosen from hydroxymethylcelluloses, hydroxyethylcelluloses and hydroxypropylcelluloses.

13. Composition according to any one of the preceding claims, in which the celluloses are celluloses modified with a hydrophobic group, it bering possible for these celluloses to be cationic or non-cationic.

14. Composition according to any one of the receding claims, **characterized in that** the cellulose derivatives are quaternized celluloses comprising at least one (C₈-C₃₀) fatty chain and chosen from the group made up of celluloses and hydroxyethylcelluloses which are quaternized and modified with alkyl or arylalkyl or alkylaryl groups comprising from 8 to 30 carbon atoms, or mixtures thereof.

15. Composition according to any one of the preceding claims, comprising a coupler chosen from meta-phenylenediamines, meta-aminophenols, meta-diphenols, naphthalene couplers, heterocyclic coupleurs, and addition salts thereof.

16. Process for oxidation dyeing of keratin fibres, **characterized in that** a dyeing composition as defined in any one of Claims 1 to 15 is applied to the fibres in the presence of an oxidizing agent for a time sufficient to develop the desired colouring.

17. Multicompartment device, in which a first compartment contains a dyeing composition as defined tin any one of Claims 1 to 15 and a second compartment contains an oxidizing agent.
